# EUROPEAN PATENT SPECIFICATION

(11) **EP 3 898 992 B1**
(45) Date of publication and mention of the grant of the patent: **24.05.2023**
(21) Application number: 19820801.9
(22) Date of filing: 18.12.2019
(51) Int. Cl.: C12N 15/85

(54) **HYBRID PROMOTERS AND THEIR USES IN THERAPY, NOTABLY FOR TREATING TYPE II COLLAGENOPATHIES**
HYBRIDE PROMOTOREN UND DEREN VERWENDUNGEN IN DER THERAPIE, INSBESONDERE ZUR BEHANDLUNG VON TYP-II-KOLLAGENOPATHIEN
PROMOTEURS HYBRIDES ET LEURS UTILISATIONS EN THÉRAPIE, NOTAMMENT POUR LE TRAITEMENT COLLAGÉNOPATHIES DE TYPE II

(30) Priority: 19.12.2018 EP 18306740
(43) Date of publication of application: 27.10.2021
(73) Proprietor: INSTITUT NATIONAL DE LA SANTE ET DE LA RECHERCHE MEDICALE (INSERM), 75013 Paris (FR); Centre national de la recherche scientifique, 75016 Paris (FR); Université Côte d'Azur, 06100 Nice (FR)
(72) Inventor: GOUZE, Elvire, 06108 NICE CEDEX 2 (FR)
(74) Representative: Maiwald GmbH
(86) International application number: PCT/EP2019/086020
(87) International publication number: WO 2020/127533

(56) References cited:
- WO-A2-2010/062951
- US-B1- 6 448 470
- JONATHAN C. BERNHARD ET AL: "Should we use cells, biomaterials, or tissue engineering for cartilage regeneration?", STEM CELL RESEARCH & THERAPY, vol. 7, no. 1, 18 April 2016 (2016-04-18), XP055569577, DOI: 10.1186/s13287-016-0314-3
- RYAN M C ET AL: "The human type II procollagen gene: Identification of an additional protein-coding domain and location of potential regulatory sequences in the promoter and first intron", GENOMICS, ACADEMIC PRESS, SAN DIEGO, US, vol. 8, no. 1, 1 September 1990 (1990-09-01), pages 41-48, XP024864873, ISSN: 0888-7543, DOI: 10.1016/0888-7543(90)90224-I [retrieved on 1990-09-01] & Anonymous: "COL2A1 - Collagen alpha-1(II) chain precursor - Homo sapiens (Human) - COL2A1 gene & protein", , 1 January 2009 (2009-01-01), XP055569715, Retrieved from the Internet: URL:https://www.uniprot.org/uniprot/P02458 [retrieved on 2019-03-15]
- M VIKKULA ET AL: "Structural analysis of the regulatory elements of the type-II procollagen gene. Conservation of promoter and first intron sequences between human and mouse", BIOCHEMICAL JOURNAL, vol. 285, no. 1, 1 July 1992 (1992-07-01), pages 287-294, XP055569638, GB ISSN: 0264-6021, DOI: 10.1042/bj2850287
- WANG WEN ET AL: "Enhanced transgene expression using cis-acting elements combined with the EF1 promoter in a mammalian expression system", EUROPEAN JOURNAL OF PHARMACEUTICAL SCIENCES, ELSEVIER, AMSTERDAM, NL, vol. 123, 12 August 2018 (2018-08-12), pages 539-545, XP085463113, ISSN: 0928-0987, DOI: 10.1016/J.EJPS.2018.08.016
- TERESE MAGNUSSON ET AL: "Sustained, high transgene expression in liver with plasmid vectors using optimized promoter-enhancer combinations : High transgene expression in liver using promoter-enhancer combinations", JOURNAL OF GENE MEDICINE, vol. 13, no. 7-8, 1 July 2011 (2011-07-01) , pages 382-391, XP055572288, US ISSN: 1099-498X, DOI: 10.1002/jgm.1585
- Terese Magnusson ET AL: "Sustained, high transgene expression in liver with plasmid vectors using optimized promoter-enhancer combinations : High transgene expression in liver using promoter-enhancer combinations", The Journal of Gene Medicine, vol. 13, no. 7-8, 1 July 2011 (2011-07-01) , pages 382-391, XP055570094, US ISSN: 1099-498X, DOI: 10.1002/jgm.1585

## Description

### FIELD OF THE INVENTION

The present invention relates to hybrid promoters and their uses in therapy, especially in gene therapy, more particularly for treating type II collagenopathies.

### BACKGROUND OF THE INVENTION

Type II collagenopathies are a class of skeletal dysplasia that result from mutations in the type II collagen gene (COL2A1). There exists a variety of type II collagenopathies (1-4). Hypochondrogenesis and achondrogenesis have a lethal presentation and are characterized by severely underossified skeleton. Other diseases such as spondyloepiphyseal dysplasia congenita (SEDC), Kniest dysplasia or Stickler syndrome have a neonatal or childhood presentation.

Spondyloepiphyseal dysplasia congenita is the most severe non-lethal type II collagenopathy (5). SEDC has an estimated prevalence of 1/100,000 live births and an autosomal dominant transmission (6, 7). It is characterized by an abnormality in the development of collagen type II tissues, leading to short stature with very short trunk, abnormal epiphyses, flattened vertebral bodies, cleft palate, retinal degeneration and other eye abnormalities such as myopia. Hearing loss has also been reported. There is a high risk of life-threatening neck instability.

The COL2A1 gene is specifically transcribed in a limited number of tissues including articular cartilage, the cornea and the vitreous of the eye, the inner ear and the nucleus polposus of the spine (8). It encodes the α1 chain of procollagen type II. Three α1 chains are folded together on a triple-helical structure to form the procollagen homotrimer, characterized by Gly-X-Y triplets' repetition. Procollagen chains are first synthesized as propeptides containing N- and C-terminal portions. After secretion into the extracellular matrix, the terminal portions are cleaved forming the mature type II collagen that can form collagen fibrils. Collagen type II is the major fibrillar protein of hyaline cartilage and facilitates the specific arrangement of chondrocytes providing an architectural plan for the elongation of bones (9). Several mutations in the COL2A1 gene have been identified in SEDC patients and are directly responsible for the clinical features. The most common mutations are substitutions of a glycine in cysteine or serine in the Gly-X-Y triplets leading to the absence or abnormal fibrils resulting in premature degradation of the cartilage matrix (10). It is important to notice that all other enzymes necessary for the complex maturation of collagen are normal.

There is currently no cure for type II collagenopathies, and current care consists in managing and treating symptoms, including invasive surgeries (11).

To better understand the mechanisms of said pathologies, some authors tried to develop animal or cellular models. Okada et al. have generated new cellular models by converting patient fibroblasts from patients with achondrogenesis into induced chondrocytic cells or by generating iPS cells (12). In this study, they attempted to decrease endoplasmic reticulum (ER) stress or to enhance other cartilage signaling pathways such as BMP signaling, showing some potential benefit, but most importantly they developed a cellular model that will be useful for pathophysiological studies and drug screening.

WO2010/062951 relates to transgenic animals having a chondrocyte-specific Col2al promoter. US 6448471 B1 relates to transgenic COL2A1-null mice whose genomes comprise a nucleic acid sequence encoding human COL2A1 operatively linked to a promoter. Bernhard, J.C., Vunjak-Novakovic, G. "Should we use cells, biomaterials, or tissue engineering for cartilage regeneration?", Stem Cell Res Ther7, 56 (2016). https://doi.org/10.1186/s13287-016-0314-3 discloses that chondrocytes differentiated from pluripotent stem cells had higher gene expression for chondrocyte and cartilage-producing genes (COL2A1, AGC, SOX9). Ryan MC et al., "The human type II procollagen gene: identification of an additional protein-coding domain and location of potential regulatory sequences in the promoter and first intron". Genomics, 1990 Sep;8(1):41-8. doi: 10.1016/0888-7543(90)90224-I discloses the complete DNA sequence of the 5' portion of the human type II procollagen gene (COL2A1). Vikkula et al, Structural analysis of the regulatory elements of the type-II procollagen gene. Conservation of promoter and first intron sequences between human and mouse, August 1992, Biochemical Journal 285 (Pt 1)(1):287-94, DOI:10.1042/bj2850287 relates to analysis of the structural motifs of COL2AI.

Anyway, there still exists a serious and unfulfilled need for a curative treatment of type II collagenopathies.

### SUMMARY OF THE INVENTION

The subject matter of the invention is as defined in the appended claims.

The inventors have surprisingly developed a gene therapy approach to restore bone growth, particularly in SEDC. The aim is to provide the altered growth plate cartilage with normal collagen type II, thus allowing for a reorganization of the cartilage matrix which permits bone growth. Specifically, this gene therapy approach is allowed thanks to designed hybrid promoters, which are able to significantly express type II collagen in bone cells, while being specific in that they limit expression to tissues naturally expressing type II collagen.

Thus, in a first aspect, the invention relates to a promoter comprising the nucleic acid sequence SEQ ID NO:1, in which a fragment starting from position A and ending at position B of said sequence SEQ ID NO:1 has been substituted by a fragment of the hEF1α promoter comprising at least the TATA box,
wherein position A is comprised between nucleotides 521 and 555 of SEQ ID NO:1, and position B is comprised between nucleotides 560 and 587 of SEQ ID NO:1, and wherein the fragment consisting of nucleotides 598 to 607 of SEQ ID NO:1 may be present or absent, wherein the fragment of the hEF1α promoter comprising at least the TATA box is chosen from sequences SEQ ID NO:2, 3 and 4.

Such a promoter is a hybrid promoter, and is a promoter according to the invention.

In a second aspect, the invention relates to a vector comprising the promoter according to the invention.

In a third aspect, the invention relates to the use of the promoter according to the invention, or of the vector as described above, in therapy. Particularly, said use is for gene therapy, such as an ex *vivo* gene therapy or an *in vivo* gene therapy.

In a fourth aspect, the invention relates to the use of the promoter according to the invention, or of the vector as described above, for treating a skeletal dysplasia or a disease of the musculoskeletal system.

In a fifth aspect, the invention relates to the use of the promoter according to the invention, or of the vector as described above, for treating a type II collagenopathy.

### DETAILED DESCRIPTION OF THE INVENTION

As shown in the example, the inventors have surprisingly developed a gene therapy to restore bone growth, particularly in the case of type II collagenopathies such as SEDC. Such an approach is very surprising, because the complex synthesis of type II collagen must be performed at the site of the disease (i.e. in the chondrocytes). On the other hand, it is known that the viruses have a very limited penetration into the hyaline cartilage matrix.

Surprisingly, the inventors have been successful in preparing a specific hybrid promoter which was integrated into a vector in which it controls the expression of the pro-alpha1(II) chain of type II collagen (encoded by COL2A1 gene), and said vector is able to penetrate into the growth plate. This was not expected: hyaline cartilage and growth cartilage are two separate and different structures, and the vector which comprises the hybrid promoter is able to penetrate into the growth plate, whereas it is not able to penetrate the hyaline cartilage.

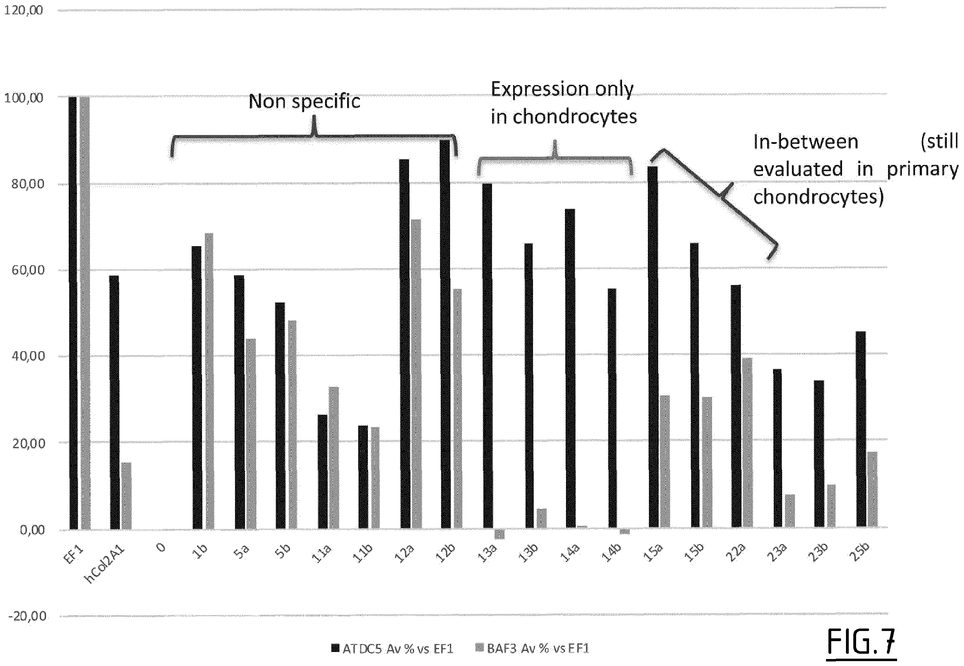

Moreover, once injected into cells, said vector induces the specific expression of type II collagen by chondrocytes as compared to other cells such as B lymphocytes; it is thus tissue specific (as shown in Figure 7). Furthermore, said expression is high in chondrocytes.

After *in vivo* injection of said vector into a murine model of type II collagenopathy, a significant increase of survival is observed. This increased survival is associated with a restoration of the rib cage volume and of the foramen magnum area, and with a correction of the position of the skull following treatment. Moreover, body and long bones growth increases are observed. Thus, the developed vector constitutes a promising tool useful in therapy, particularly gene therapy. Indeed, said developed vector may be useful for increasing body length, for increasing long bones, for increasing the rib cage volume, for increasing the foramen magnum area, for correcting the position of the cervical vertebra and/or for reorganizing the structure of the growth plate of long bones.

Finally, the inventors generated induced Pluripotent Stem Cells (iPSCs) from patients with a SEDC mutation, and were able to demonstrate that loss of COL2A1 impaired mesenchymal stem cell (MSCs) differentiation, associated with a decrease in expression of cell surface markers specific of MSCs in Sedc iPSCs compared to control cells. Treatment with a vector comprising a hydrid promoter according to the invention and COL2A1 gene was able to restore MSC markers expression level similar to that of control cells. The presence of only 10% of treated cells was sufficient to restore a proper mesenchymal differentiation.

In a first aspect, the invention thus relates to a promoter comprising the nucleic acid sequence SEQ ID NO:1, in which a fragment starting from position A and ending at position B of said sequence SEQ ID NO:1 has been substituted by a fragment of the hEF1α promoter comprising at least the TATA box,
wherein position A is comprised between nucleotides 521 and 555 of SEQ ID NO:1, and position B is comprised between nucleotides 560 and 587 of SEQ ID NO:1, and
wherein the fragment consisting of nucleotides 598 to 607 of SEQ ID NO:1 may be present or absent.
Of course, the promoter of the invention is different from SEQ ID NO:1.

SEQ ID NO:1 is the whole sequence of the promoter of human COL2A1 gene (hCOL2A1).

COL2A1 is the gene coding for the α1 chain of procollagen type II (which is also called the pro-alpha1(II) chain of type II collagen). The sequence of said human α1 chain of procollagen type II protein may be found in Uniprot under accession number P02458. To construct type II collagen, three pro-alpha1 (II) chains twist together to form a triple-stranded, rope-like procollagen molecule. Procollagen molecules are then processed by enzymes in the cell. Once processed, the molecules leave the cell and arrange themselves into long, thin fibrils that link to one another (cross-link) in the spaces around cells. The cross-linkages result in the formation of very strong, mature type II collagen fibers.

The term "promoter" refers to a nucleic acid sequence located upstream or 5' to a translational start codon of an open reading frame (or protein-coding region) of a gene and that is involved in recognition and binding of RNA polymerase II and other proteins (trans-acting transcription factors) to initiate transcription. A "hybrid promoter" is a non-native promoter that is functional in host cells, particularly in mammal cells such as human cells. Indeed, the hybrid promoter of the invention comprises a part of the sequence of the promoter of hCOL2A1 gene, but also at least the TATA box of the hEF1α promoter.

The different sequences which are listed in this application are as follows:

| **SEQ ID NO:** | **Nature of the sequence** |
|---|---|
| 1 | Full-length sequence of the promoter of hCOL2A1 (nucleotides 1-716) |
| 2 | Fragment of the hEF1α promoter which starts from EGFP1 and which ends after the TATA box ("EGFP1-linker-EGFP2-TATA box") |
| | (nucleotides 104 to 180 of SEQ ID NO:5) |
| 3 | Fragment of the hEF1α promoter which only consists in the TATA box ("TATA box") |
| | Said sequence is TATATAA |
| 4 | Fragment of the hEF1α promoter which comprises the TATA box, its 5' (upstream) sequence and a part of its 3' (downstream) sequence ("--TATA box-") |
| | (nucleotides 154 to 213 of SEQ ID NO:5) |
| 5 | Full-length sequence of the promoter of hEF1α |
| 6 | Fragment of the hCOL2A1 promoter which starts from the pyrimidine-rich sequence P and which ends after the second enhancer core element E ("P-E-S-S-N1-AP-2ε-N1-S-S-E"). It is called "hybrid promoter 1" on figure 5 |
| | (nucleotides 226 to 520 of SEQ ID NO:1) |
| 7 | Fragment of the hCOL2A1 promoter which starts from the 5' N1 sequence and which ends after the first 3' SP1-binding site S ("N1-AP-2ε-N1-S"). It is called "hybrid promoter 5" on figure 5 |
| 8 | Sequence including the N1 sequence in 3' of the hCOL2A1 promoter. |
| | Said sequence is GGGCTGCCGG |
| 9 | EGFP1 of the hEF1α promoter |
| 10 | EGFP2 of the hEF1α promoter |
| 11 | Hybrid promoter 13a |
| 12 | Hybrid promoter 13b |
| 13 | Hybrid promoter 14a |
| 14 | Hybrid promoter 14b |
| 15 | Hybrid promoter 15a |
| 16 | Hybrid promoter 15b |
| 17 | Hybrid promoter 22a |
| 18 | Hybrid promoter 1a |
| 19 | Hybrid promoter 1b |
| 20 | Hybrid promoter 5a |
| 21 | Hybrid promoter 5b |
| 22 | Hybrid promoter 11a |
| 23 | Hybrid promoter 11b |
| 24 | Hybrid promoter 12a |
| 25 | Hybrid promoter 12b |
| 26 | Hybrid promoter 23a |
| 27 | Hybrid promoter 24a |
| 28 | Hybrid promoter 25a |
| 29 | Hybrid promoter 25b |
| 30 | Hybrid promoter 23b |

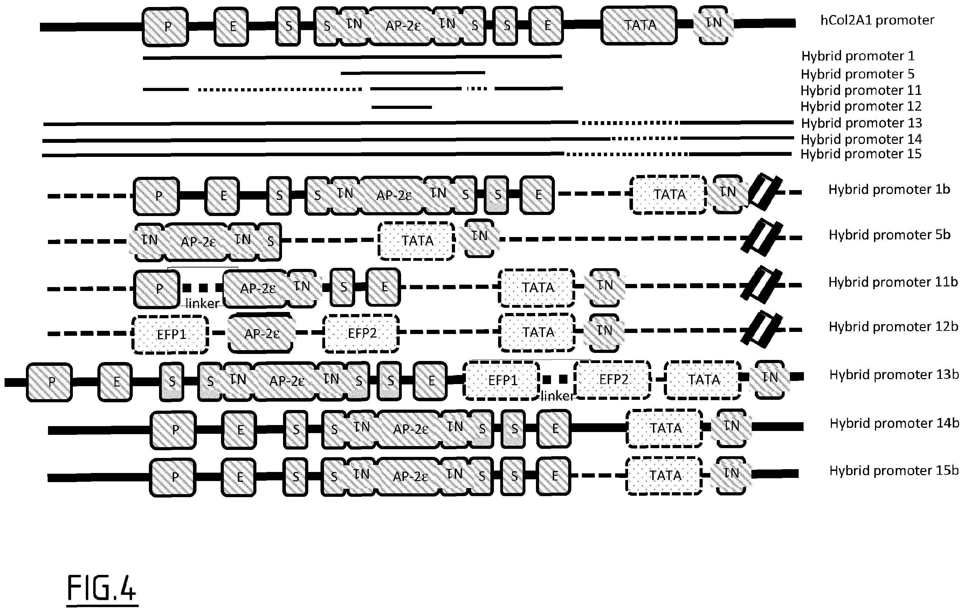

As indicated in figure 4, the hCOL2A1 promoter comprises different elements, which are:
- the TATA box,
- SP1-binding sites (S),
- enhancer core elements (E),
- a pyrimidine-rich sequence (P),
- the AP-2ε site, which corresponds to the fixation of AP2 transcription factor, which is specific of cartilage, and
- N1 sequences. These sequences are cis-regulatory elements (CREs) which bind to chondrocyte nuclear proteins. These proteins are repressive factors. There are three N1 sequences in native hCOL2A1 promoter: two N1 sequences are framing the AP-2ε site, and the last N1 sequence is in 3'.

The core sequence of the hCOL2A1 promoter corresponds to SEQ ID NO:6. It comprises the pyrimidine-rich sequence P, the four SP1-binding sites (SP1), the two enhancer core elements (E) and the AP-2ε site which is in the middle of two N1 sequences. Said core sequence corresponds to nucleotides 226 to 520 of SEQ ID NO:1.

Nucleotides 1 to 520 correspond to the upstream sequence (5') and the core sequence of the hCOL2A1 promoter.

Nucleotides 521 to 555 of SEQ ID NO:1 correspond to the sequence of the hCOL2A1 promoter which is between the last enhancer core element (E) in 3' and the TATA box.

Nucleotides 556 to 560 of SEQ ID NO:1 correspond to the TATA box of the hCOL2A1 promoter (TATAA).

Nucleotides 561 to 587 of SEQ ID NO:1 correspond to the sequence of the hCOL2A1 promoter downstream (in 3') of the TATA box and upstream of the last N1 sequence.

Nucleotides 598 to 607 of SEQ ID NO:1 correspond to the sequence SEQ ID NO:8, i.e. which includes the last N1 sequence in 3' of SEQ ID NO:1.

SEQ ID NO:5 is the whole sequence of the promoter of human EF1α gene (hEF1α).

EF1α is a gene coding for the eukaryotic translation elongation factor-1 alpha 1, which is ubiquitously expressed. Its promoter is a strong positive promoter.

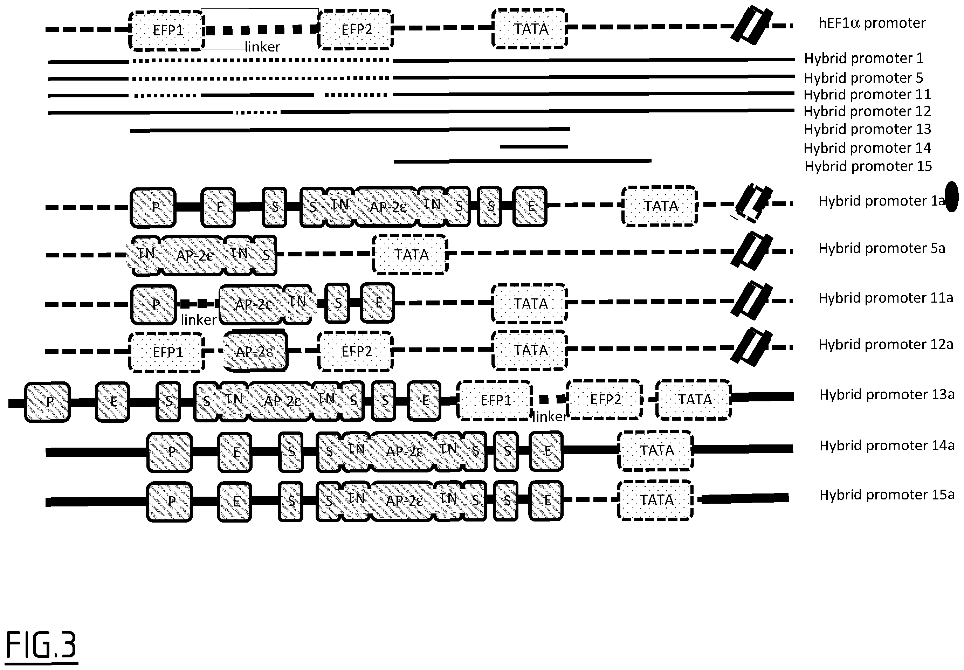

As indicated in figure 3, the hEF1α promoter comprises:
- the TATA box (which is of SEQ ID NO:3), and
- EFP1 and EFP2 elements (respectively of SEQ ID NO:9 and 10), which are known to regulate EF1α expression.

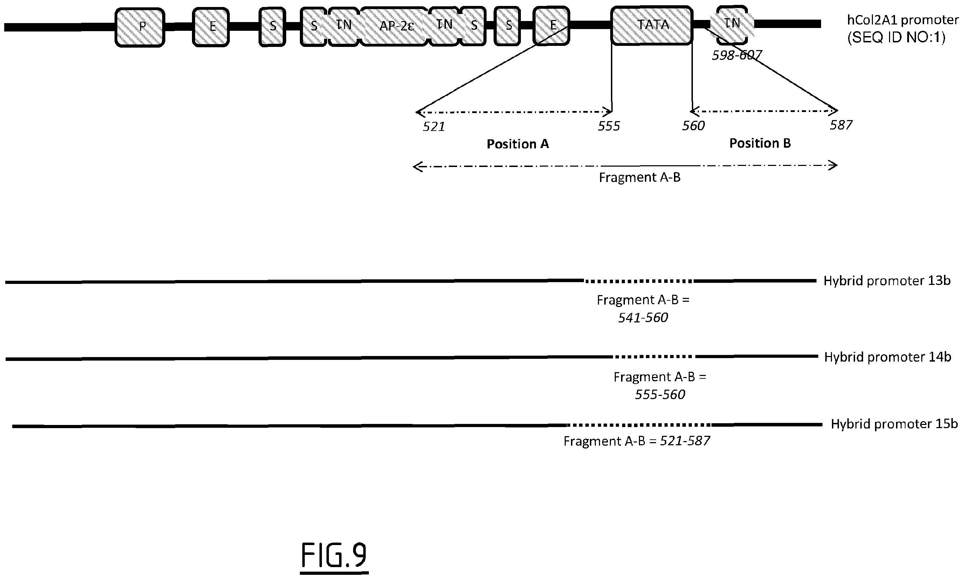

As indicated in figure 9, the hybrid promoter of the invention comprises the nucleic acid sequence of hCOL2A1 promoter (SEQ ID NO:1), in which a fragment thereof has been substituted by a fragment of the hEF1α promoter comprising at least the TATA box.

The fragment of hCOL2A1 promoter which is taken from SEQ ID NO:1 is defined by its position (i.e. between positions A and B) in SEQ ID NO:1. It starts from position A and ends at position B. Thus, of course, positions A and B are included in the fragment. Position A is comprised between nucleotides 521 and 555 of SEQ ID NO:1, and position B is comprised between nucleotides 560 and 587 of SEQ ID NO:1.

Preferably, position A is at nucleotide 521, 541 or 555 of SEQ ID NO:1, and position B is at nucleotide 560 or 587 of SEQ ID NO:1.

In other words, the hybrid promoters of the invention comprise SEQ ID NO:1 in which a fragment A-B has been removed and substituted by a fragment of the hEF1α promoter comprising at least the TATA box.

Moreover, in the hybrid promoter of the invention, the fragment consisting of nucleotides 598 to 607 of SEQ ID NO:1 may be present or absent. As indicated above, said fragment is SEQ ID NO:8 and corresponds to the sequence including the N1 sequence in 3' of the hCOL2A1 promoter.

When said fragment consisting of nucleotides 598 to 607 of SEQ ID NO:1 is present, the corresponding promoters are called version "b" in the present application.

When said fragment consisting of nucleotides 598 to 607 of SEQ ID NO:1 is absent, the corresponding promoters are called version "a" in the present application.

According to an embodiment of the invention, the fragment of the hEF1α promoter comprising at least the TATA box is chosen from:
- the fragment consisting of nucleotides 104 to 180 of SEQ ID NO:5;
- the fragment consisting of nucleotides 174 to 180 of SEQ ID NO:5; and
- the fragment consisting of nucleotides 154 to 213 of SEQ ID NO:5.

Preferably, the fragment of the hEF1α promoter comprising at least the TATA box is chosen from sequences SEQ ID NO:2, 3 and 4.

According to a preferred embodiment of the invention, the fragment starting from nucleotide 541 and ending at nucleotide 560 of sequence SEQ ID NO:1 has been substituted by the fragment (of the hEF1α promoter comprising at least the TATA box) of sequence SEQ ID NO:2, and the fragment consisting of nucleotides 598 to 607 of SEQ ID NO:1 is absent. As indicated in figure 3, the corresponding hybrid promoter is the promoter 13a.

Hybrid promoter 13a according to the invention has the sequence SEQ ID NO:11.

Alternatively, according to a preferred embodiment of the invention, the fragment starting from nucleotide 541 and ending at nucleotide 560 of sequence SEQ ID NO:1 has been substituted by the fragment (of the hEF1α promoter comprising at least the TATA box) of sequence SEQ ID NO:2, and the fragment consisting of nucleotides 598 to 607 of SEQ ID NO:1 is present. As indicated in figures 4 and 9, the corresponding hybrid promoter is the promoter 13b.

Hybrid promoter 13b according to the invention has the sequence SEQ ID NO:12.

Alternatively, according to a preferred embodiment of the invention, the fragment starting from nucleotide 555 and ending at nucleotide 560 of sequence SEQ ID NO:1 has been substituted by the fragment of the hEF1α promoter comprising at least the TATA box of sequence SEQ ID NO:3, and the fragment consisting of nucleotides 598 to 607 of SEQ ID NO:1 is absent. As indicated in figure 3, the corresponding hybrid promoter is the promoter 14a.

Hybrid promoter 14a according to the invention has the sequence SEQ ID NO:13.

Alternatively, according to a preferred embodiment of the invention, the fragment starting from nucleotide 555 and ending at nucleotide 560 of sequence SEQ ID NO:1 has been substituted by the fragment (of the hEF1α promoter comprising at least the TATA box) of sequence SEQ ID NO:3, and the fragment consisting of nucleotides 598 to 607 of SEQ ID NO:1 is present. As indicated in figures 4 and 9, the corresponding hybrid promoter is the promoter 14b.

Hybrid promoter 14b according to the invention has the sequence SEQ ID NO:14.

Alternatively, according to a preferred embodiment of the invention, the fragment starting from nucleotide 521 and ending at nucleotide 587 of sequence SEQ ID NO:1 has been substituted by the fragment (of the hEF1α promoter comprising at least the TATA box) of sequence SEQ ID NO:4, and the fragment consisting of nucleotides 598 to 607 of SEQ ID NO:1 is absent. As indicated in figure 3, the corresponding hybrid promoter is the promoter 15a.

Hybrid promoter 15a according to the invention has the sequence SEQ ID NO:15.

Alternatively, according to a preferred embodiment of the invention, the fragment starting from nucleotide 521 and ending at nucleotide 587 of sequence SEQ ID NO:1 has been substituted by the fragment (of the hEF1α promoter comprising at least the TATA box) of sequence SEQ ID NO:4, and the fragment consisting of nucleotides 598 to 607 of SEQ ID NO:1 is present. As indicated in figures 4 and 9, the corresponding hybrid promoter is the promoter 15b.

Hybrid promoter 15b according to the invention has the sequence SEQ ID NO:16.

Further disclosed is a promoter comprising the nucleic acid sequence SEQ ID NO: 17 or SEQ ID NO:29, or comprising the nucleic acid sequence SEQ ID NO:5, in which the sequence SEQ ID NO: 7 has been inserted between nucleotides 89 and 90 of said sequence SEQ ID NO:5, and the fragment starting from nucleotide 104 and ending at nucleotide 153 of said sequence SEQ ID NO:5 has been substituted by the sequence SEQ ID NO:7, and optionally the sequence SEQ ID NO:8 has been inserted between nucleotides 217 and 218 of SEQ ID NO:5.

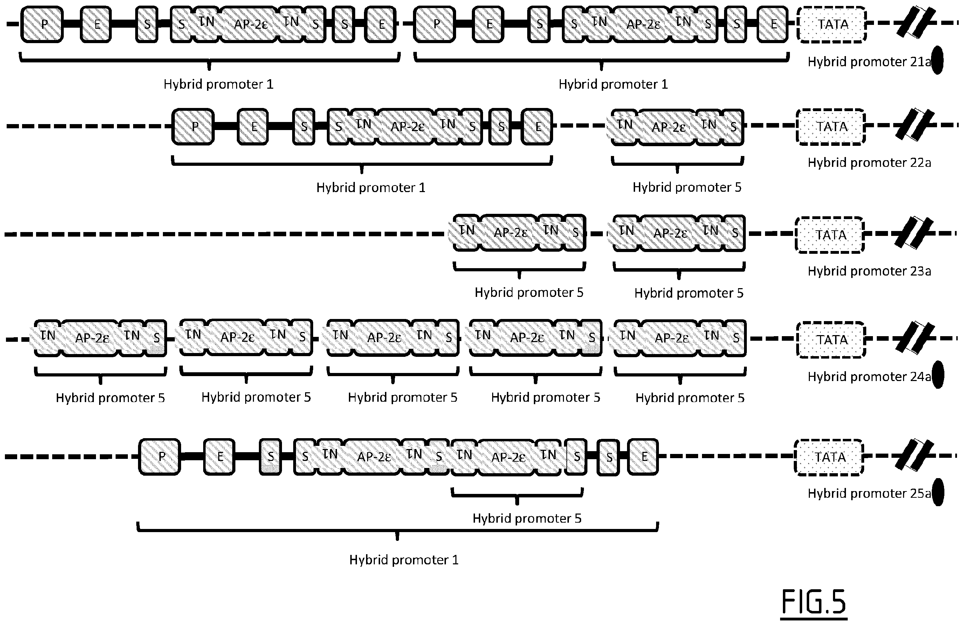

Further disclosed is a promoter comprising the nucleic acid sequence SEQ ID NO:17. As indicated in figure 5, the corresponding hybrid promoter is the promoter 22a.

According to another aspect, the present invention relates to a promoter comprising the nucleic acid sequence SEQ ID NO:5, in which the sequence SEQ ID NO: 7 has been inserted between nucleotides 89 and 90 of said sequence SEQ ID NO:5, and the fragment starting from nucleotide 104 and ending at nucleotide 153 of said sequence SEQ ID NO:5 has been substituted by the sequence SEQ ID NO:7. As indicated in figure 5, the corresponding hybrid promoter is the promoter 23a. Said promoter has preferably the sequence SEQ ID NO:26.

According to another aspect, the present invention relates to a promoter comprising the nucleic acid sequence SEQ ID NO:5, in which:
- the sequence SEQ ID NO: 7 has been inserted between nucleotides 89 and 90 of said sequence SEQ ID NO:5,
- the fragment starting from nucleotide 104 and ending at nucleotide 153 of said sequence SEQ ID NO:5 has been substituted by the sequence SEQ ID NO:7; and
- the sequence SEQ ID NO:8 has been inserted between nucleotides 217 and 218 of SEQ ID NO:5.

As indicated in figure 5, the corresponding hybrid promoter is the promoter 23b. Said promoter has preferably the sequence SEQ ID NO:30.

According to another aspect, the present invention relates to a promoter comprising the nucleic acid sequence SEQ ID NO:29. As indicated in figure 5, the corresponding hybrid promoter is the promoter 25b.

Preferably, the hybrid promoter is chosen from promoters 13a, 13b, 14a, 14b, 15a, 15b and 22a. More preferably, the hybrid promoter is chosen from promoters 13a, 13b, 14a, 14b, 15a and 15b. More preferably, the hybrid promoter is chosen form promoters 13a, 13b, 14a and 14b.

According to an embodiment, the hybrid promoter of the invention controls the expression of a nucleic acid sequence of interest. The "nucleic acid sequence of interest" may be a RNA or DNA sequence (including cDNA sequences), preferably a gene. Preferably, the nucleic acid sequence of interest is operably linked to the hybrid promoter of the invention. The phrase "operably linked" means that the nucleic acid sequence of interest is linked downstream of the promoter so that said nucleic acid sequence of interest is transcribed according to the promoter activity.

Preferably, the nucleic acid sequence of interest is a gene or a fragment thereof coding for a collagen chain. Preferably, it is a human gene or fragment thereof coding for a collagen chain. Said gene or fragment thereof may be native or recombinant. The collagen chain may be of any one of types I to XXVIII. Preferably, the collagen chain is of type I (i.e. encoded by COL1A1 or COL1A2 genes), or of type II (i.e. encoded by COL2A1 gene). More preferably, the collagen chain is of type II. More preferably, the collagen chain is a human collagen chain. More preferably, the collagen chain is the pro-alpha1 (II) chain of type II collagen, preferably the human one (encoded by hCOL2A1 gene).

COL2A1 gene is expressed in the articular cartilage, the cornea and the vitreous of the eye, the inner ear and the nucleus polposus of the spine.

The present invention also relates to a vector comprising a promoter according to the invention.

As used herein, the term "vector" refers to a polynucleotide which is capable of being introduced or of introducing the nucleic acid sequence of interest into a cell. In the context of the present invention, it is preferred that the nucleic acid sequence of interest is expressed within the cell upon introduction of the vector. Both viral and non-viral vector systems can be used. Suitable vectors are known in the art and include, for example, plasmids, artificial chromosomes (e.g. bacterial, mammalian such as human, or yeast aritificial), virus or phage vectors provided with an origin of replication, and optionally a regulator of the promoter. Vectors may be used to transfect or transform a host cell, for example, a mammalian host cell, e.g., for the production of the nucleic acid sequence of interest encoded by the vector. The vectors may also be adapted to be used *in vivo,* for example, in a method of vaccination or gene therapy.

Examples of suitable viral vectors include retroviral, lentiviral, adenoviral, adeno-associated viral, herpes viral, including herpes simplex viral, alpha-viral, pox viral, such as Canarypox and vaccinia-viral based systems. Gene transfer techniques using these viruses are known in the art. Retrovirus vectors, for example, may be used to stably integrate the nucleic acid sequence of interest into the host genome. Replication-defective adenovirus vectors by contrast remain episomal and therefore allow transient expression. Vectors capable of driving expression in insect cells (e.g., baculovirus vectors), in human cells, yeast, or in bacteria may be employed in order to produce quantities of the nucleic acid sequence of interest, for example, for use in subunit vaccines or in immunoassays. In an additional example, a replication-deficient simian adenovirus vector may be used as a live vector. These viruses contain an E1 deletion and can be grown on cell lines that are transformed with an E1 gene. These vectors can be manipulated to insert a nucleic acid sequence of interest, such that the encoded protein may be expressed.

According to a preferred embodiment, the vector is a viral vector, and more preferably a lentiviral vector or an adeno-associated virus (AAV).

Preferably, the vector comprises a hybrid promoter of the invention which controls the expression of a gene or a fragment thereof coding for a collagen chain, preferably the pro-alpha1(II) chain of type II collagen.

### Therapeutic uses

The present invention also relates to the use of a hybrid promoter of the invention, or to the use of a vector comprising the hybrid promoter according to the invention in therapy. Preferably, the therapy is gene therapy. Said gene therapy may be an ex *vivo* gene therapy. *Ex vivo* gene therapy consists in transfecting a vector comprising the hybrid promoter according to the invention into isolated cells, and thereafter transplanting the transfected cells into the patient. Useful cells which may be of interest are mesenchymal stem cells (MSC) cells or cells removed from the patient himself. Alternatively, said gene therapy may be an *in vivo* gene therapy. *In vivo* gene therapy comprises injecting a vector comprising the hybrid promoter according to the invention directly into the patient.

The present invention further relates to a method for treating a subject in need thereof, which comprises administering to said subject a vector comprising the hybrid promoter according to the invention.

As used herein, the term "subject" or "patient" denotes a human or non-human mammal, such as a rodent, a feline, a canine, an equine, or a primate. Preferably, the subject is a human being, more preferably a child (i.e. in terms of the present invention a human that is still growing, particularly in height). Indeed, as indicated previously, the hybrid promoter and vector according to the invention are able to penetrate into the cartilaginous matrix of the growth plate, and/or in cells which are annex to cartilage such as perichondrium.

In one embodiment, the subject has been diagnosed as suffering from a skeletal dysplasia as described herein. In one embodiment, the subject has been diagnosed as suffering from an asphyxiating syndrome as described herein.

In another embodiment, the subject has been diagnosed as suffering from a type II collagenopathy as described herein.

In another embodiment, the subject has been diagnosed as suffering from a disease of the musculoskeletal system as described herein.

As used herein, "treat", "treating", or "treatment" of a disease or disorder means accomplishing one or more of the following: (a) reducing the severity of the disorder; (b) limiting or preventing development of symptoms characteristic of the disorder(s) being treated; (c) inhibiting worsening of symptoms characteristic of the disorder(s) being treated; (d) limiting or preventing recurrence of the disorder(s) in an individual that have previously had the disorder(s); and (e) limiting or preventing recurrence of symptoms in individuals that were previously symptomatic for the disorder(s).

The vector comprising the hybrid promoter according to the invention is preferably delivered through gene therapy, where it is delivered to tissues of interest and expressed in vivo. Gene therapy methods are discussed, e.g., in Verme et al. (Nature 389:239-242, 1997), Yamamoto et al. (Molecular Therapy 1 7:S67-S68, 2009), and Yamamoto et al., (J. Bone Miner. Res. 26: 135-142, 201 1).

Expression regulatory signals may be selected to be compatible with the host cell for which expression is designed.

The introduced nucleic acid sequence of interest can be stably or transiently maintained in the host cell. Stable maintenance typically requires that the introduced nucleic acid sequence of interest either contains an origin of replication compatible with the host cell or integrates into a replicon of the host cell such as an extrachromosomal replicon (e.g., a plasmid) or a nuclear or mitochondrial chromosome.

According to an embodiment, the present invention further relates to the use of a hybrid promoter of the invention, or to the use of a vector comprising the hybrid promoter according to the invention, for treating a skeletal dysplasia or a disease of the musculoskeletal system.

According to an embodiment, the present invention further relates to the use of a hybrid promoter of the invention, or to the use of a vector comprising the hybrid promoter according to the invention, for treating a type II collagenopathy.

The term "skeletal dysplasia" or "osteochondrodysplasia", as used herein, refers to a heritable group of more than 450 well delineated disorders that affect primarily bone and cartilage, but can also have significant effects on muscle, tendons and ligaments. By definition, skeletal dysplasias are heritable diseases that have generalized abnormalities in cartilage and bone.

Examples of skeletal dysplasia include spondyloepiphyseal dysplasia congenita (SEDC), Kniest dysplasia, Stickler syndrome, Jeune syndrome (or asphyxiating thoracic dystrophy), achondroplasia, homozygous achondroplasia, heterozygous achondroplasia, achondrogenesis, acrodysostosis, acromesomelic dysplasia, atelosteogenesis, camptomelic dysplasia, chondrodysplasia punctata, rhizomelic type of chondrodysplasia punctata, cleidocranial dysostosis, congenital short femur, craniosynostosis (e.g., Muenke syndrome, Crouzon syndrome, Apert syndrome, Jackson-Weiss syndrome, Pfeiffer syndrome, or Crouzonodermoskeletal syndrome), dactyly, brachydactyly, camptodactyly, Polydactyly, syndactyly, diastrophic dysplasia, dwarfism, dyssegmental dysplasia, enchondromatosis, fibrochondrogenesis, fibrous dysplasia, hereditary multiple exostoses, hypochondroplasia, hypophosphatasia, hypophosphatemic rickets, Jaffe-Lichtenstein syndrome, Langer-type mesomelic dysplasia, Marfan syndrome, McCune-Albright syndrome, micromelia, metaphyseal dysplasia, Jansen-type metaphyseal dysplasia, metatrophic dysplasia, Morquio syndrome, Nievergelt-type mesomelic dysplasia, neurofibromatosis (e.g. type 1, e.g., with bone manifestations or without bone manifestations; type 2; schwannomatosis; or any described herein), osteochondrodysplasia, osteogenesis imperfecta, perinatal lethal type of osteogenesis imperfecta, osteopetrosis, osteopoikilosis, peripheral dysostosis, Reinhardt syndrome, Roberts syndrome, Robinow syndrome, short-rib Polydactyly syndromes, short stature, spondyloepimetaphyseal dysplasia, thanatophoric dysplasia, spondylocostal dysostosis, pseudorheumatoid dysplasia, all the aggrecanopathies, or Leri Weil dyschondrosteosis.

Accordingly, the invention provides for the treatment of a skeletal dysplasia, such as notably spondyloepiphyseal dysplasia congenita (SEDC), Kniest dysplasia, Stickler syndrome or Jeune syndrome.

The term "type II collagenopathy", as used herein, refers to a group of genetic disorders that affect connective tissue, and which are caused by defects in type II collagen. Type II collagenopathies are a subtype of skeletal dysplasia. For example, type II collagenopathies may include spondyloepiphyseal dysplasia congenita (SEDC), Kniest dysplasia, Stickler syndrome, achondrogenesis type 2, hypochondrogenesis, Czech dysplasia, Legg-Calvé-Perthes disease, platyspondylic lethal skeletal dysplasia of Torrance type or spondyloepimetaphyseal dysplasia of Strudwick type.

Accordingly, the invention provides for the treatment of a type II collagenopathy, and preferably spondyloepiphyseal dysplasia congenita (SEDC), Kniest dysplasia or Stickler syndrome.

The term "disease of the musculoskeletal system", as used herein, refers to a skeletal disease which affects joints. For example, the articular pathologies include arthritis, and in particular osteoarthritis, rheumatoid arthritis, gout, septic arthritis, ankylosing spondylitis, juvenile idiopathic arthritis or Still's disease.

Provided herein are notably methods for treating a skeletal dysplasia in a patient, such as a patient (like a human) having a type II collagenopathy such as spondyloepiphyseal dysplasia congenita. In particular, the patient may exhibit or may be likely to have one or more symptoms of a skeletal dysplasia (e.g., a type II collagenopathy such as spondyloepiphyseal dysplasia congenita). The method involves administering a vector comprising a hybrid promoter of the invention operably linked to a nucleic acid sequence of interest, such as a sequence coding for a collagen chain (such as the pro-alpha1(II) chain of type II collagen, encoded by COL2A1 gene), or a fragment thereof, to the patient (e.g., a human). For example, the patient exhibits signs or symptoms of a skeletal dysplasia, such as those described herein (e.g., a type II collagenopathy such as spondyloepiphyseal dysplasia congenita), e.g., prior to administration of the vector. Treatment with a vector of the invention can also occur after a patient (e.g., a human) has been diagnosed with a skeletal dysplasia, such as those described herein (e.g., a type II collagenopathy such as spondyloepiphyseal dysplasia congenita), or after a patient exhibits signs or symptoms of a skeletal dysplasia, such as those described herein (e.g., a type II collagenopathy such as spondyloepiphyseal dysplasia congenita).

Treatment with a vector comprising the hybrid promoter according to the invention can result in an improvement in a symptom of a skeletal dysplasia, e.g., a type II collagenopathy such as spondyloepiphyseal dysplasia congenita. The methods can be used to treat symptoms associated with a skeletal dysplasia, e.g., a type II collagenopathy such as spondyloepiphyseal dysplasia congenita, such that there is reversal or a reduction in the severity of symptoms of the skeletal dysplasia, e.g., a type II collagenopathy such as spondyloepiphyseal dysplasia congenita.

Any skeletal dysplasia that is a type II collagenopathy (e.g., caused by or associated with a mutation in COL2A1 gene) can be treated by administering a vector comprising a hybrid promoter of the invention operably linked to a sequence coding for a collagen chain, such as the pro-alpha1 (II) chain of type II collagen encoded by COL2A1 gene, as described herein to a patient (e.g., a human). For example, such a vector can be administered to treat a type II collagenopathy such as spondyloepiphyseal dysplasia congenita.

Administration of such a vector may be used to treat the type II collagenopathies resulting from any one of the R275C, G546S, R437P, R740C, R789C, G897R mutations of the pro-alpha1(II) chain of type II collagen encoded by COL2A1 gene, as well as any one of the mutations of COL2A1 gene causing Stickler syndrome, such as the C57Y, G216D, G219R, G222V, G240D, G267D, G270R, G282D, del302-308, G453A, G492D, G501R, R565C, R904C, G1131A or G1158A mutations. Especially, the type II collagenopathy may be spondyloepiphyseal dysplasia congenita (SEDC). Preferably, the type II collagenopathy may be due to a glycine substitution.

Thus, a patient treated with the vector described in the above paragraph may have a mutation in the COL2A1 gene.

Any of the aforementioned mutations in the COL2A1 gene can be detected in a sample from the patient (e.g., a human with a type II collagenopathy such as spondyloepiphyseal dysplasia congenita) prior to or after treatment (e.g., treatment with the vector described herein). Additionally, the parents of the patient and/or fetal samples (e.g., fetal nucleic acid obtained from maternal blood, placental, or fetal samples) may be tested by methods known in the art for the mutation.

Administration of a vector as disclosed herein may result in an improvement in symptoms including, but not limited to, growth retardation, skull deformities, orthodontic defects, cervical cord compression (with risk of death, e.g., from central apnea or seizures), spinal stenosis (e.g., leg and lower back pain), hydrocephalus (e.g., requiring cerebral shunt surgery), hearing loss due to chronic otitis, cardiovascular disease, neurological disease, respiratory problems, fatigue, pain, numbness in the lower back and/or spin, and obesity.

Patients treated using the vector described herein may include, e.g., infants, children, and adults with a skeletal dysplasia or disease of the musculoskeletal system. Infants are often diagnosed with type II collagenopathies at birth, and thus, treatment with a vector as described herein, may begin as early as possible in the patient's life, e.g., shortly after birth, or prior to birth (in utero).

Symptoms of skeletal dysplasia (e.g., type II collagenopathy) or disease of the musculoskeletal system in patients (e.g., humans) may also be monitored prior to or after a patient is treated with a vector comprising the hybrid promoter according to the invention. For instance, the symptoms may be monitored prior to treatment to assess the severity of the disease and condition of the patient prior to performing the methods. The methods of the invention may include diagnosis of the disease in a patient and monitoring the patient for changes in the symptoms of the disease, such as changes in body weight, body height, sitting height, skull shape, skull length and/or skull width but also cervical instability, respiratory complications and/or neurological complications of the patient based on changes monitored over a period of time, e.g., 1, 2, 3, 4 or more times per month or per year or approximately every 1, 2, 3, 4, 5, 6, 7, 8, 12 or 16 weeks over the course of treatment with the vector comprising the hybrid promoter of the present invention. Body weight and/or skull size of the patient or changes thereof can also be determined at treatment specific events, e.g. before and/or after administration of the vector comprising the hybrid promoter according to the invention. For example, body weight and/or skull size are measured in response to administration of the vector of the present invention.

Body weight can be measured simply be weighing the subject on a scale, preferably in a standardized manner, e.g. with the same (in particular for humans) or no clothes or at a certain time of the day, preferably in a fasting state (for example in the morning before breakfast is taken, or after at least 1, 2, 3, 4, 5 or more hours of fasting).

Skull size is preferably represented by length, height, width and/or circumference.

Measurements can be taken by any known or self-devised standardized method. For a human subject, the measurement of skull circumference is preferred. It is usually taken with a flexible and non-stretchable material such as a tape, which is wrapped around the widest possible circumference of the head (though not around the ears or the facial area below and including the eyebrows), e.g. from the most prominent part of the forehead around to the widest part of the back of the head. Another preferred measurement for a human subject can determine the height of the skull, for example from the underside of the chin to the uppermost point of the head. For a rodent subject, the measurement of the length of the skull (e.g. tip of the nasal bone to back of the occipital bone) is preferred.

Alternatively, also the width of the skull (e.g. widest points of the parietal bone), the height of the skull (e.g. lowest point of the angular process of lower jaw to frontal bone) or the volume of the skull, are preferred. Preferably, any measurement is taken more than once, e.g. at least 3 times, and the largest number is taken as the length, height, width and/or circumference.

Preferably, in case of human subjects, the symptoms of the disease to be considered are typically changes in body weight, body height, sitting height, skull shape, long bone length (such as tibia length, humerus length and/or femur length), cervical instability, respiratory complications and/or neurological complications.

### Pharmaceutical compositions and formulations

A composition of the present invention (e.g., including a vector comprising the hybrid promoter according to the invention) can be administered by a variety of methods known in the art. As will be appreciated by the skilled artisan, the route and/or mode of administration will vary depending upon the desired results. The route of administration can depend on a variety of factors, such as the environment and therapeutic goals. In particular, the vector comprising at least one hybrid promoter of the invention, can be administered by any route known in the art, e.g., subcutaneous (e.g., by subcutaneous injection), intravenously, orally, nasally, intramuscularly, sublingually, intrathecal or intradermally. By way of example, pharmaceutical compositions of the invention can be in the form of a liquid, solution, suspension, pill, capsule, tablet, gel cap, powder, gel, ointment, cream, nebulae, mist, atomized vapor, aerosol or phytosome.

Any amount of a pharmaceutical composition (including a vector comprising the hybrid promoter according to the invention) can be administered to a patient, such as a patient with a skeletal dysplasia (e.g., a patient with a type II collagenopathy such as spondyloepiphyseal dysplasia congenita). The dosages will depend on many factors including the mode of administration and the age of the patient. Typically, the amount of the composition (e.g., including a vector according to the invention) can be an amount that is effective to treat a condition (e.g., a type II collagenopathy such as spondyloepiphyseal dysplasia congenita) as described herein without inducing significant toxicity.

For example, the vector according to the invention described herein can be administered to patients (e.g., patients with a type II collagenopathy) in individual doses ranging, e.g., from 0.0002 mg/kg to about 20 mg/kg, e.g., from 0.002 mg/kg to 20 mg/kg, from 0.01 mg/kg to 2 mg/kg, from 0.02 mg/kg to 20 mg/kg, from 0.01 mg/kg to 10 mg/kg, from 10 mg/kg to 100 mg/kg, from 0.1 mg/kg to 50 mg/kg, 0.5 mg/kg to 20 mg/kg, 1.0 mg/kg to 10 mg/kg, 1.5 mg/kg to 5 mg/kg or 0.2 mg/kg to 3 mg/kg. In particular, the vector comprising the hybrid promoter according to the invention can be administered in individual doses of, e.g., 0.001 mg/kg to 7 mg/kg. These doses can be administered one or more times (e.g., 2, 3, 4, 5, 6, 7, 8, 9, 10, 11 or 12 or more times) per day, week, month, or year.

Preferably, at least one dose, preferably at least two doses, preferably at least three doses, of the composition comprising the vector of the invention, can be an amount that is effective to treat a condition (e.g., a type II collagenopathy such as spondyloepiphyseal dysplasia congenita) as described herein. Preferably, the composition comprising the vector of the invention may be administered once a week.

Exemplary doses of the composition comprising the vector comprising the hybrid promoter according to the invention include, e.g., 0.0002, 0.0005, 0.001, 0.002, 0.005, 0.01, 0.02, 0.05, 0.1, 0.2, 0.5, 1, 2, 3, 4, 5, 6, 7, 8, 9, 10, 15 or 20 mg/kg. For all dosages or ranges recited herein, the term "about" may be used to modify these dosages by ±10% of the recited values or range endpoints. In particular, the composition comprising the vector comprising the hybrid promoter according to the invention can be administered to patients in doses ranging from about 0.0002 mg/kg/day to about 20 mg/kg/day, about 0.02 mg/kg/day to about 15 mg/kg/day, or about 0.2 mg/kg/day to about 10 mg/kg/day (e.g., 0.75 mg/kg/day). For example, the composition comprising the vector of the invention can be administered to patients in a weekly dosage ranging, e.g., from about 0.0014 mg/kg/week to about 140 mg/kg/week, e.g., about 0.14 mg/kg/week to about 105 mg/kg/week, or, e.g., about 1.4 mg/kg/week to about 70 mg/kg/week (e.g., 5 mg/kg/week). The dosage will be adapted by the clinician in accordance with conventional factors such as the extent of the disease and different parameters from the patient.

Dosages of compositions comprising the vector comprising the hybrid promoter according to the invention may be provided in either a single or multiple dosage regimens. Doses can be administered, e.g., hourly, bihourly, daily, bi-daily, twice a week, three times a week, four times a week, five times a week, six times a week, weekly, biweekly, monthly, bimonthly, or yearly.

Alternatively, doses can be administered, e.g., twice, three times, four times, five times, six times, seven times, eight times, nine times, 10 times, 11 times or 12 times per day.

The amount, frequency, and duration of dosage will be adapted by the clinician in accordance with conventional factors such as the extent of the disease and different parameters from the patient.

### Carriers/vehicles

Compositions comprising the vector of the invention may be provided to patients in combination with pharmaceutically acceptable sterile aqueous or non-aqueous solvents, suspensions or emulsions. Examples of non-aqueous solvents are propylene glycol, polyethylene glycol, vegetable oil, fish oil, and injectable organic esters. Aqueous carriers include water, water-alcohol solutions, emulsions or suspensions, including saline and buffered medical parenteral vehicles including sodium chloride solution, Ringer's dextrose solution, dextrose plus sodium chloride solution, Ringer's solution containing lactose, or fixed oils.

Intravenous vehicles may include fluid and nutrient replenishers, electrolyte replenishers, such as those based upon Ringer's dextrose, and the like. Pharmaceutically acceptable salts can be included therein, for example, mineral acid salts such as hydrochlorides, hydrobromides, phosphates, sulfates, and the like; and the salts of organic acids such as acetates, propionates, malonates, benzoates, and the like. Additionally, auxiliary substances, such as wetting or emulsifying agents, pH buffering substances, and the like, may be present in such vehicles. A thorough discussion of pharmaceutically acceptable carriers is available in Remington's Pharmaceutical Sciences (Mack Pub. Co., N.J. 1991).

Preferably, the pharmaceutical compositions contain vehicles which are pharmaceutically acceptable for a formulation capable of being injected. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions. The doses used for the administration can be adapted as a function of various parameters, and in particular as a function of the mode of administration used, of the relevant pathology, or alternatively of the desired duration of treatment. For example, it is well within the skill of the art to start doses of the compound at levels lower than those required to achieve the desired therapeutic effect and to gradually increase the dosage until the desired effect is achieved. However, the daily dosage of the products may be varied over a wide range from 0.01 to 1000 mg per adult per day. Preferably, the compositions contain 0.01, 0.05, 0.1, 0.5, 1, 2.5, 5, 10, 15, 25, 50, 100, 250 and 500 mg of the active ingredient for the symptomatic adjustment of the dosage to the subject to be treated. A medicament typically contains from about 0.01 mg to about 500 mg of the active ingredient, preferably from 1 mg to about 100 mg of the active ingredient. An effective amount of the drug is ordinarily supplied at a dosage level from 0.0002 mg/kg to about 20 mg/kg of body weight per day, especially from about 0.001 mg/kg to 7 mg/kg of body weight per day. To prepare pharmaceutical compositions, an effective amount of a polypeptide according to the invention may be dissolved or dispersed in a pharmaceutically acceptable carrier or aqueous medium.

The pharmaceutical forms suitable for injectable use include sterile aqueous solutions or dispersions; formulations including sesame oil, peanut oil or aqueous propylene glycol; and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms, such as bacteria and fungi. Solutions of the active compounds as free base or pharmacologically acceptable salts can be prepared in water suitably mixed with a surfactant, such as hydroxypropylcellulose. Dispersions can also be prepared in glycerol, liquid polyethylene glycols, or mixtures thereof and in oils. Under ordinary conditions of storage and use, these preparations contain a preservative to prevent the growth of microorganisms.

The vector comprising the hybrid promoter according to the invention can be formulated into a composition in a neutral or salt form. Pharmaceutically acceptable salts include the acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like. The carrier can also be a solvent or dispersion medium containing, for example, water, ethanol, polyol (for example, glycerol, propylene glycol, and liquid polyethylene glycol and the like), suitable mixtures thereof, and vegetable oils. The proper fluidity can be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminium monostearate and gelatin.

Sterile injectable solutions are prepared by incorporating the active compounds in the required amount in the appropriate solvent with several of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof. The preparation of more, or highly concentrated solutions for direct injection is also contemplated, where the use of DMSO as solvent is envisioned to result in extremely rapid penetration, delivering high concentrations of the active agents to a small tumor area. Upon formulation, solutions will be administered in a manner compatible with the dosage formulation and in such amount as is therapeutically effective. The formulations are easily administered in a variety of dosage forms, such as the type of injectable solutions described above, but drug release capsules and the like can also be employed.

For parenteral administration in an aqueous solution, for example, the solution may be suitably buffered and the liquid diluent first rendered isotonic with sufficient saline or glucose. These particular aqueous solutions are especially suitable for intravenous, intramuscular, subcutaneous and intraperitoneal administration. In this connection, sterile aqueous media which can be employed will be known to those of skill in the art in light of the present disclosure. For example, one dosage could be dissolved in 1 ml of isotonic NaCl solution and either added to 1000 ml of a specific fluid or injected at the proposed site of infusion. Some variation in dosage will necessarily occur depending on the condition of the subject being treated. The person responsible for administration will, in any event, determine the appropriate dose for the subject.

The invention is described by way of the following examples which are to be construed as merely illustrative and not limitative of the scope of the invention.

### FIGURES

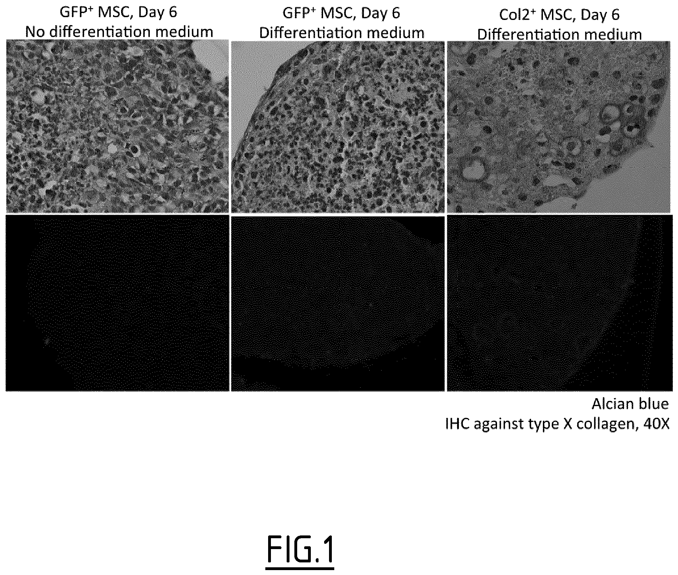

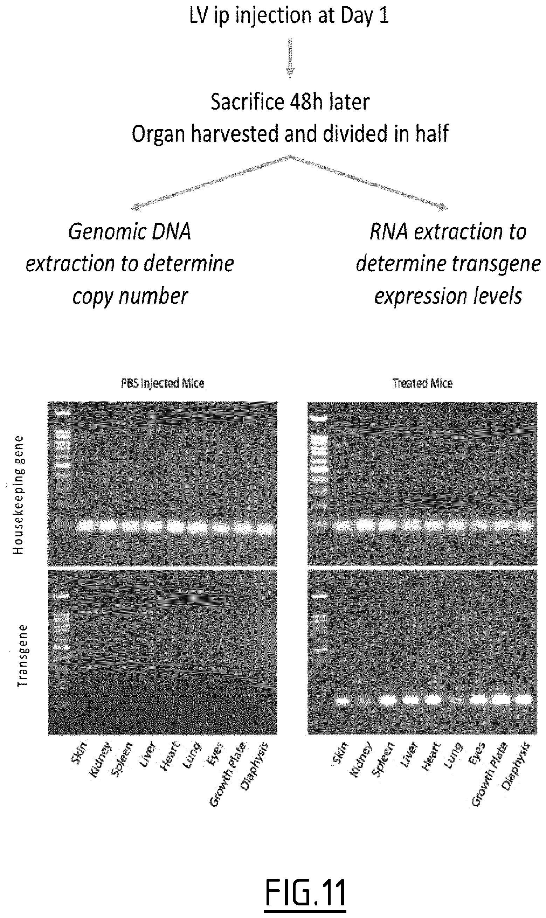

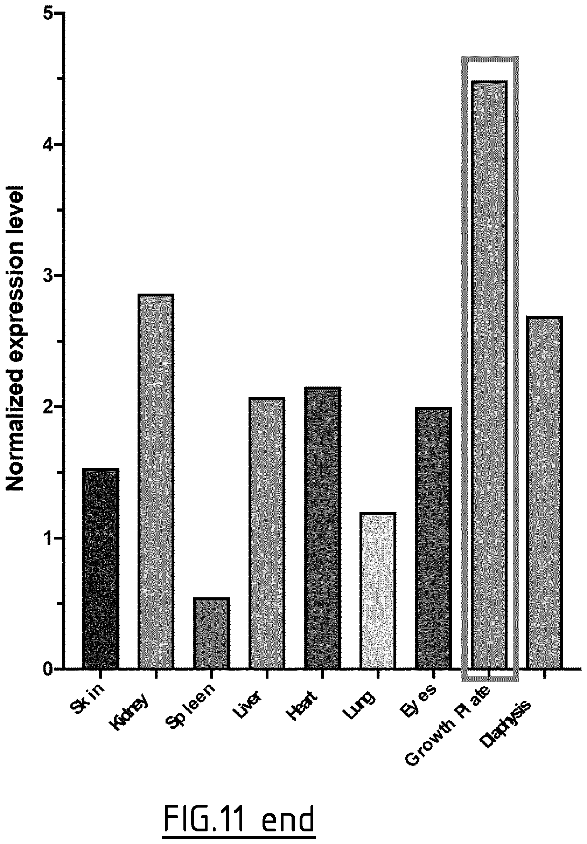

Figure 1. Evaluation of the rapidity of chondrogenic differentiation of MSCs isolated from **Col2a1^{sedc/+}** mice.
   Cells were genetically modified using a MMLV-Col2 or MMLV-GFP used as a control, in presence of chondrogenic differentiation medium. Following 6 days of culture in 3D spheroids, cartilage matrix formation was evaluated by alcian blue staining for proteoglycan content or type X collagen (in red) a marker of mature hypertrophic chondrocytes.
Figure 2. *Ex vivo* proof-of-concept of the use of an ex *vivo* gene therapy approach to restore bone growth from **Col2a1^{sedc/sedc}** mice.
   Fetal femurs from homozygous SEDC were co-cultured for five days with MSCs overexpressing Col2 or GFP. Bones were measured (a) and maturation was evaluated by Sirius red staining visualized under polarized light. The arrow indicates the presence of collagen fibers.
**Figures 3 to 6****. Schematic representation of the different hybrid promoter constructs evaluated in the study.**
   All the tested hybrid promoters (24) are illustrated.
   The promoters of the invention (such as promoters 13a, 13b, 14a, 14b, 15a, 15b and 22a) were designed to limit expression to tissues naturally expressing type II collagen.
   Around 24 constructs were designed and screened for level and specificity of expression in cellular system.
   In these figures, parts of the hEF1α promoter are in dotted lines, and EFP1, AFP2 and TATA box are in dotted boxes.
   Parts of hCOL2A1 promoter are in continuous line, and the different elements (P, E, S, N1, AP-2ε and TATA box) are in hatches.
   Hybrid promoters 1a and 1b have a hEF1α promoter backbone, and only comprise the "P-E-S-S-N1-AP-2ε-N1-S-S-E" sequence of hCOL2A1 promoter (the b version further comprises a supplementary N1 element of hCOL2A1 promoter).
   Hybrid promoters 5a and 5b have a hEF1α promoter backbone, and only comprise the "N1-AP-2ε-N1-S" sequence of hCOL2A1 promoter (the b version further comprises a supplementary N1 element of hCOL2A1 promoter).
   Hybrid promoters 11a and 11b have a hEF1α promoter backbone, and only comprise the P element and the "AP-2ε-N1-S-E" sequence of hCOL2A1 promoter (the b version further comprises a supplementary N1 element of hCOL2A1 promoter).
   Hybrid promoters 12a and 12b have a hEF1α promoter backbone, and only comprise the AP-2ε sequence of hCOL2A1 promoter (the b version further comprises a supplementary N1 element of hCOL2A1 promoter).
   Hybrid promoters 13a and 13b have a hCOL2A1 promoter backbone, and only comprise the "EFP1-linker-EFP2-TATA box" sequence of hEF1α promoter (the a version does not comprise the supplementary N1 element of hCOL2A1 promoter in 3').
   Hybrid promoters 14a and 14b have a hCOL2A1 promoter backbone, and only comprise the TATA box sequence of hEF1α promoter (the a version does not comprise the supplementary N1 element of hCOL2A1 promoter in 3').
   Hybrid promoters 15a and 15b have a hCOL2A1 promoter backbone, and only comprise the upstream 5' sequence of the TATA box, the TATA box and a part of the 3' sequence of hEF1α promoter (the a version does not comprise the supplementary N1 element of hCOL2A1 promoter in 3').
   Hybrid promoters 21a and 21b have a hEF1α promoter backbone, and only comprise twice the "P-E-S-S-N1-AP-2ε-N1-S-S-E" sequence of hCOL2A1 promoter (the b version further comprises a supplementary N1 element of hCOL2A1 promoter).
   Hybrid promoters 22a and 22b have a hEF1α promoter backbone, and only comprise the "P-E-S-S-N1-AP-2ε-N1-S-S-E" sequence and the "N1-AP-2ε-N1-S" sequence of hCOL2A1 promoter (the b version further comprises a supplementary N1 element of hCOL2A1 promoter).
   Hybrid promoters 23a and 23b have a hEF1α promoter backbone, and only comprise twice the "N1-AP-2ε-N1-S" sequence of hCOL2A1 promoter (the b version further comprises a supplementary N1 element of hCOL2A1 promoter).
   Hybrid promoters 24a and 24b have a hEF1α promoter backbone, and only comprise five times the "N1-AP-2ε-N1-S" sequence of hCOL2A1 promoter (the b version further comprises a supplementary N1 element of hCOL2A1 promoter).
   Hybrid promoters 25a and 25b have a hEF1α promoter backbone, and only comprise a mixed sequence of "P-E-S-S-N1-AP-2ε-N1-S-S-E" and "N1-AP-2ε-N1-S" of hCOL2A1 promoter (the b version further comprises a supplementary N1 element of hCOL2A1 promoter).
Figure 7. Comparative levels of expression of the different promoter constructs in Chondrocytic cells (ATDC5) or in control Baf3 cells.
   Expression is expressed in percentage of the expression levels obtained with the EF1α promoter.
   Said figure is related to **Figure 11****: Biodistribution study following injection in newborn animals.**

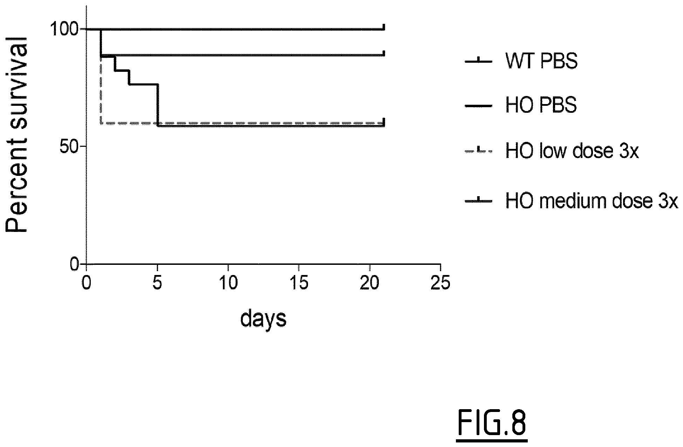

**Figure 8****. Kaplan meyer curves showing the percent survival in treated or control homozygous (HO) mice.**
**Figure 9****. Scheme of hybrid promoters of hCOL2A1 (SEQ ID NO:1) according to the invention.**
   Said promoters are derived from SEQ ID NO:1. Positions A and B are indicated on the figure. The fragment A-B is substituted by a fragment of the hEF1α promoter comprising at least the TATA box. The position of fragment A-B is given as an illustration for hybrid promoters 13b, 14b and 15b (which all comprise the N1 sequences, i.e. nucleotides 598-607).

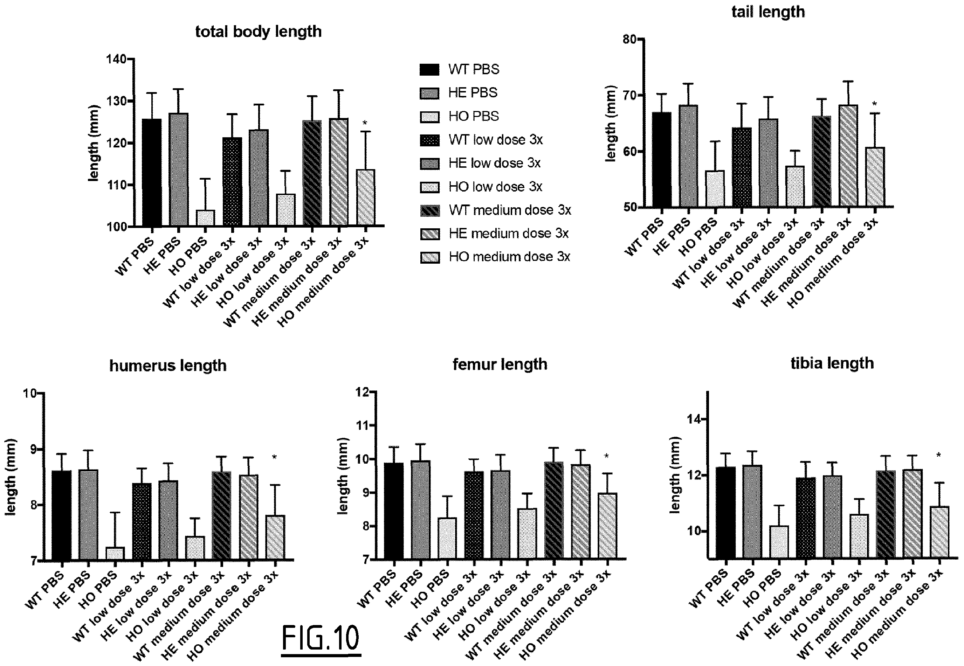

**Figure 10****. Results of the total body length, tail length, humerus length, femur length and tibia length, obtained for different experiments.**
   Mice treated during the growth period (from day 1 to day 21) by 3 injections of virus showed a dose dependent increase in overall skeletal growth, as seen by body and tail lengths, as well as the different long bones (* p< 0.05 compared to untreated HO mice). Low dose corresponds to 2×10⁵ infectious particles per ml, and medium dose to 6×10⁶ infectious particles/ml.

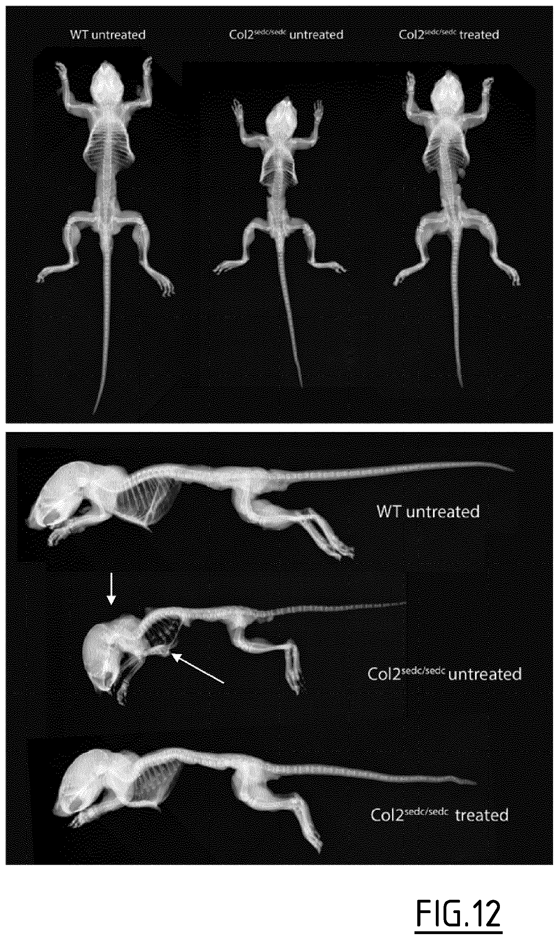

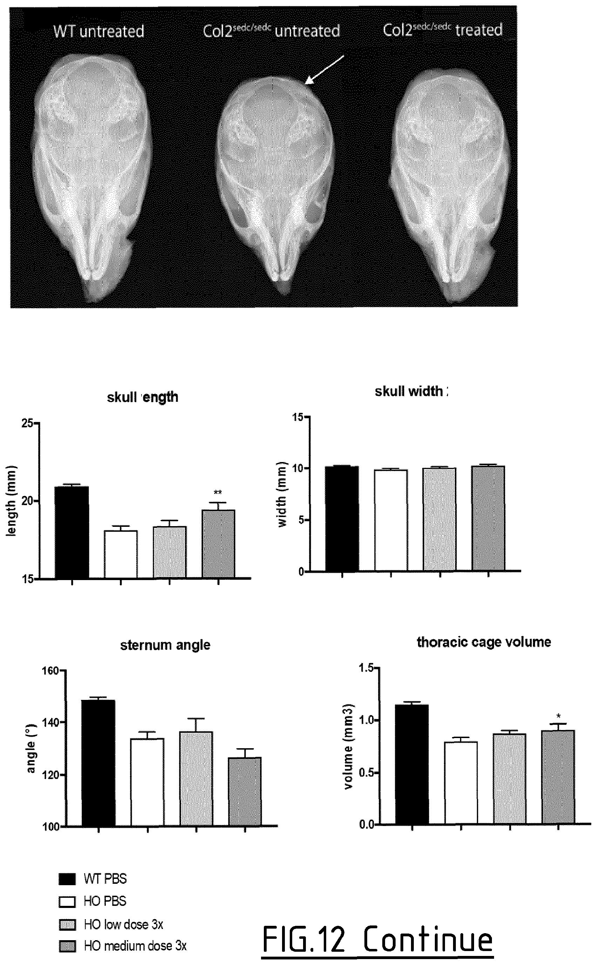

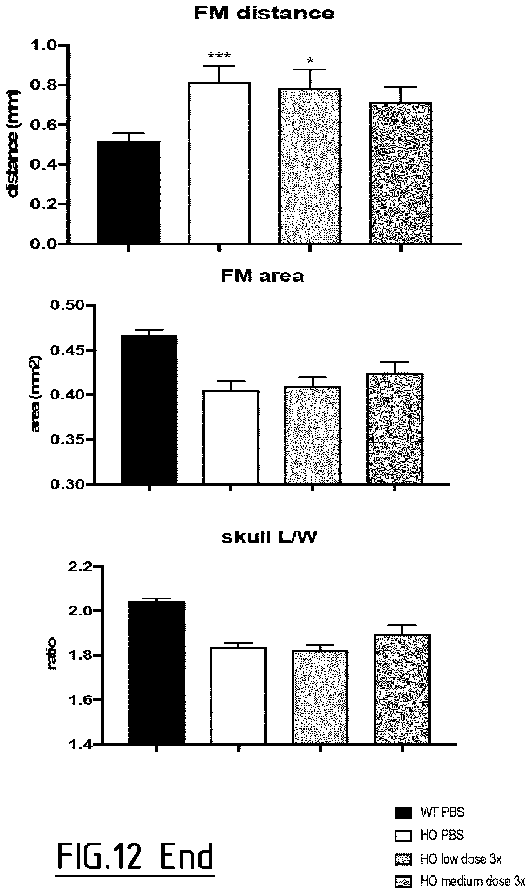

**Figure 12****. Treatment effect on the position of the foramen magnum and the thoracic cage volume.**
   Mice treated during the growth period (from day 1 to day 21) by 3 injections of virus showed a dose dependent increase in the distance between the foramen magnum and the edge of the skull and od the thoracic cage volume (* p< 0.05 compared to untreated HO mice).

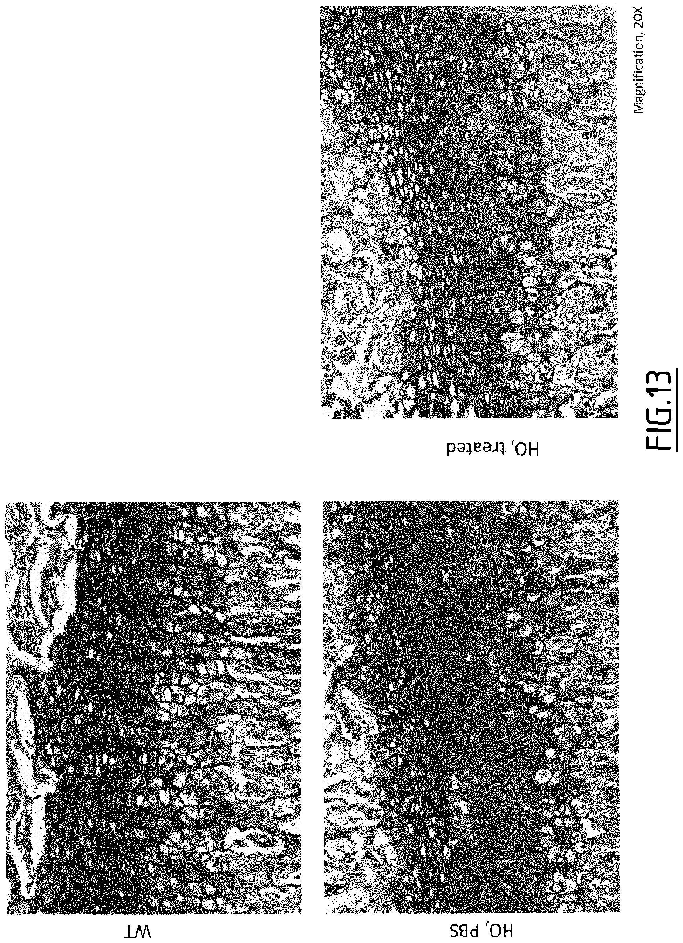

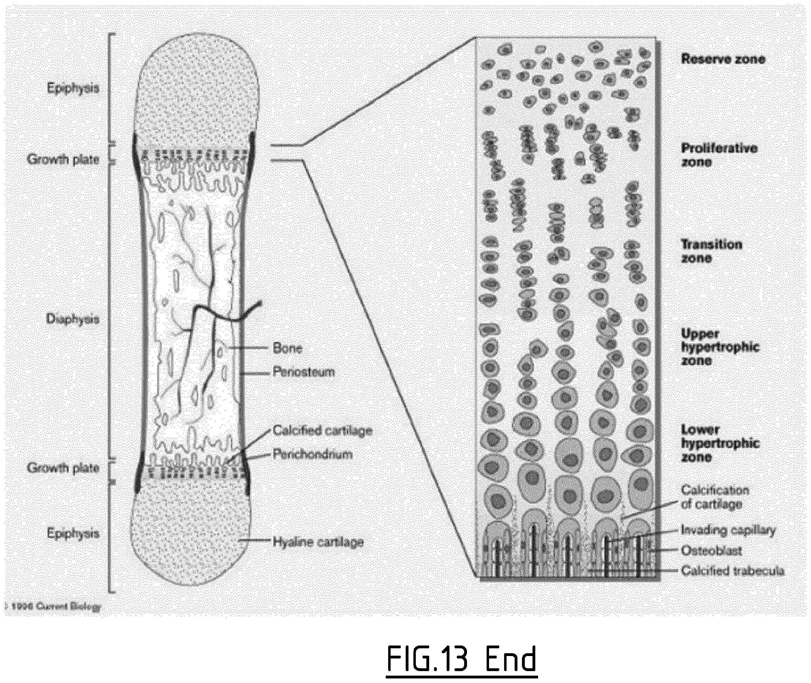

**Figure 13****. Representative images of histological sections of tibial growth plates from treated and untreated animals.**
   Immunohistochemistry against type 2 collagen is not shown, but representative images show increased expression in treated mice vs controls.

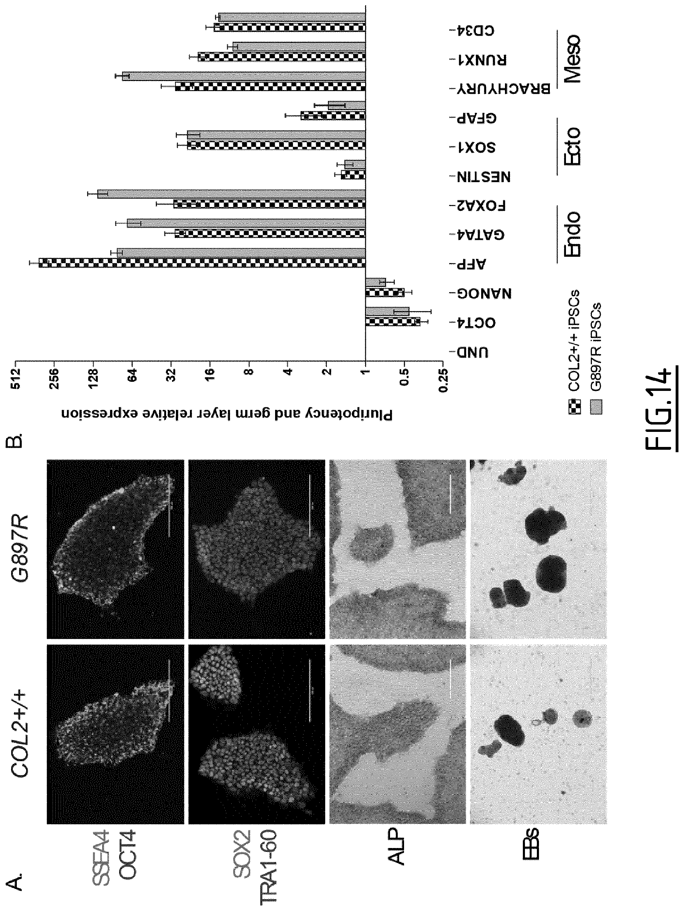

**Figure 14****. Generation of COL2^{+/+} and G897R iPSCs.**
   (A) Representative immunofluorescence staining for pluripotent markers SSEA4, OCT4, SOX2, and TRA-1-60 demonstrated that COL2+/+ and G897R iPSCs were positive for these markers. The generated iPSCs also displayed a strong alkaline phosphatase activity. Lastly, iPSCs from both genotypes are able to form EBs. One iPSCs clone is presented for each genotype.
   (B) Expression of pluripotent (*OCT4*, *NANOG*), endoderm (Endo., *AFP, GATA4, FOXA2*), ectoderm (Ecto., *NESTIN, GFAP, SOX1*) and mesoderm (Meso., *BRACHYURY, RUNX1,* CD34) gene markers for COL2+/+ (black square), G897R (plain gray) iPSCs relative to undifferentiated cells (UND). Data are represented as pooled mean ± SEM of three experiments on three clones from each genotype.

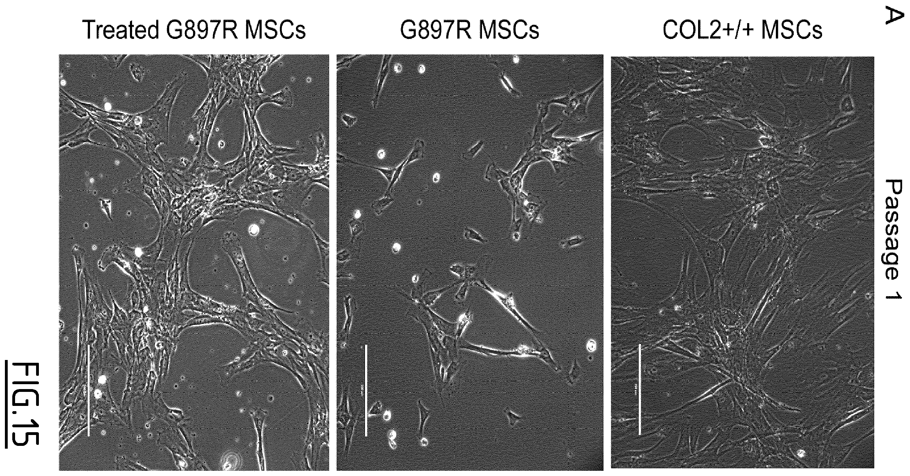

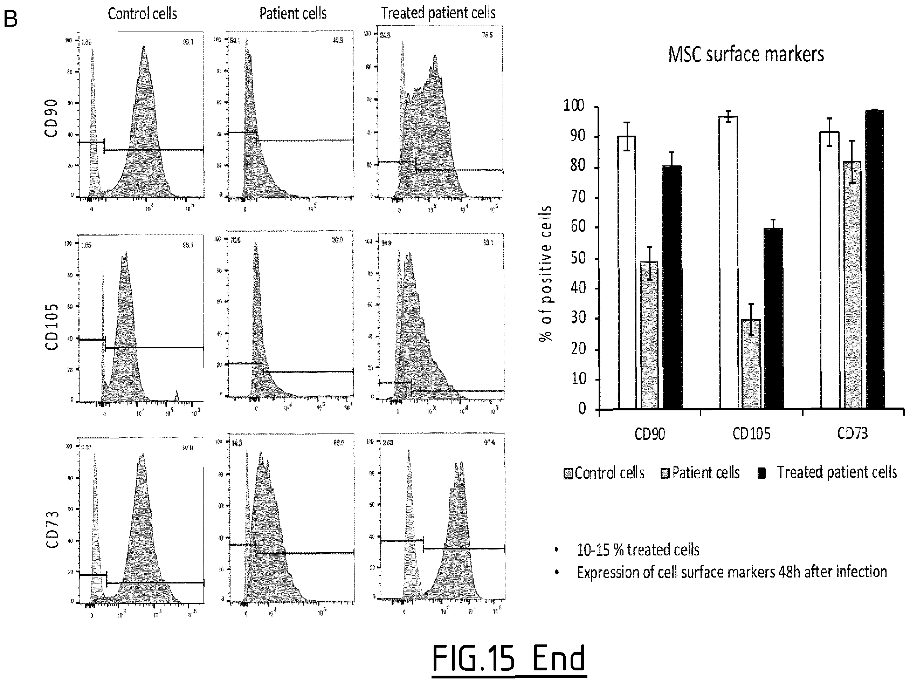

**Figure 15****. Treatment effect on the differentiation into mesenchymal stem cells from Sedc iPSCs.**
   Mesenchymal stem cells were obtained from iPSCs from control or patient and were genetically modified by the lentiviral vector. Cells were visualized under light microscopy (A) or analyzed by flow cytometry for specific cell surface markers (B).

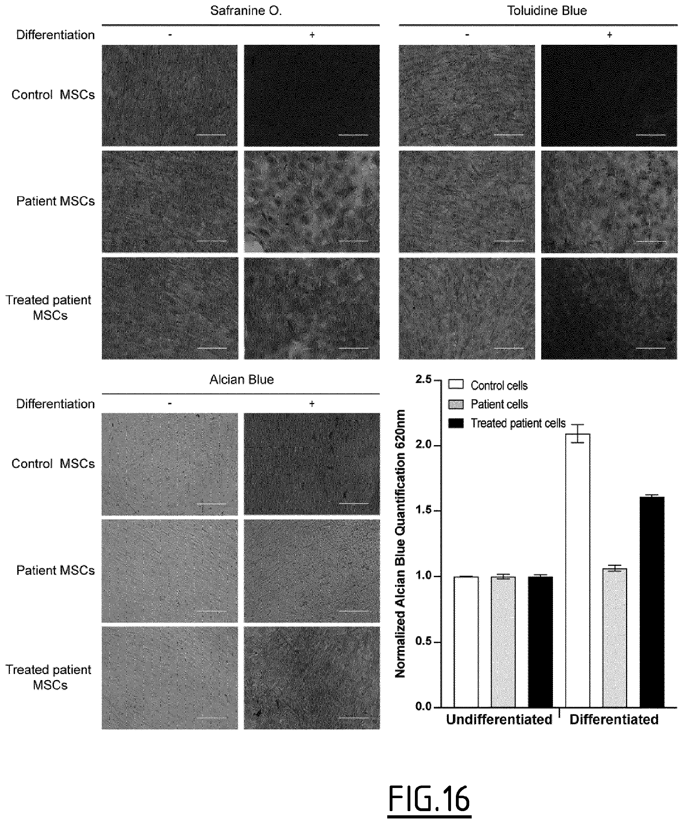

**Figure 16****. Mesenchymal stem cells isolated from Sedc patient showed a significant reduction in the production of the extracellular matrix of differentiated chondrocytes that was restored by the gene therapy.**

### EXAMPLE

### Material and Methods

### 1- Lentiviral particle production and characterization

Lentiviral particles are produced by transient transfection of 3 plasmids using 293T cells in Milliczll HY T-600 flasks (Millipore). When reaching 70% confluence, cells are transfected using a VSV-G envelope plasmid, a human PAX2 expression vector and the plasmid containing the hybrid promoter and gene of interest (hCOL2A1). Experiments were performed using an expression cassette containing the human COL2A1 gene followed by an IRES element and the GFP genes. Transfections were performed by classical CaCl₂ transfection method. After 72h, lentiviral particles are harvested, filtered on a 40 µm cell strainer, and concentrated by ultracentrifugation.

Viral preparations are then characterized by p24 ELISA and by FACS analysis to determine the infectious particles contents.

### 2- Liquid overlay cell culture

Following isolation from 6 week old mice by femoral flushing, mesenchymal stem cells (MSCs) were culture in 3D settings using the liquid overlay cell culture method. For this, 20 000 cells were seeded in a well of a 96 well V-bottom plate by low grade centrifugation at 1000 *g* for 10 min. Spheroids formed after 24 hrs in culture. Cells were treated by the viral particles at the time of seeding. After 2-3 weeks in cultures, the spheroids were analyzed by histology.

### 3- Animal housing

A spontaneous model of mice with type II collagenopathy was used for this study (commercially available at the Jax Laboratories). These mice, B6(Cg)-Col2a1^{sedc}/GrsrJ, contain a mutation on exon 48 chromosome 15 in the COL2A1 gene. They resume essentially the human phenotype, with a shortened trunk and long bones, inner ear defects and hearing impairment. All animals were kept in a temperature and humidity-controlled room, exposed to a 12-hour light/dark cycle and had free access to standard laboratory food and water. Pups were kept with their genitors at all times. The Principles of Laboratory Animal Care (National Institutes of Health publication no. 85-23, revised 1985; http://grants1.nih.gov/grants/olaw/references/phspol.htm) and the European commission guidelines for the protection of animals used for scientific purposes (http://ec.europa.eu/environment/chemicals/lab_animals/legislation en.htm) were followed at all times. The study was approved by the local Institutional Ethic Committee for the use of Laboratory Animals (CIEPAL Azur) (approval APAFIS#13604-2018040515554889 v3).

To avoid any bias in the experimentation and stay blinded for result analysis, genotyping is performed at the end of the experiments using a small piece of ear for genomic DNA extraction.

### 4- Treatment

Col2a1^{sedc/+} female mice were bred with Col2a1^{sedc/+} or Col1a1^{sedc/sedc} mice to generate WT, heterozygous (HE) and homozygous (HO) animals. Whole litters were randomly allocated to treatment groups. Animals received at day 1 and at day 7 an intraperitoneal injection of a low dose of 1.8×10⁶ infectious particles in 10 µl of the 13a construct or of PBS. Animals were carefully observed daily to follow mortality and occurrence of complications (respiratory complications, paralysis, tail deformities, pectus excavatum). Body weight was measured every 2 days for 21 days. At day 21, animals were euthanized by a lethal injection of dolethal. Animals are carefully dissected. Organs (spleen, kidneys, liver, lung, heart) were weight and fixed in 10% formalin for 24 hrs along with retina, femoral head cap, knees and sternum for histological analysis. Total body length, tail length and cranium proportions are then measured using a digital caliper (Within 300mm). Mice skeleton are then X-rayed using a Faxitron X-ray using a 26kv exposure for 19 seconds. All long bones, vertebra, sternum characteristics, foramen magnum are then measured and analyzed using the HOROS software.

### 5- Histology and immunostaining

Following formalin fixation, tissues (3D cultures, organs, cartilage, bones) were embedded in paraffin. Bones were previously decalcified in 10% EDTA pH7.4.

5 µm sections were then stained with alcian blue or processed for immunohistochemistry for type II collagen, type X collagen or aggrecan. For this, following epitope retrieval using 10 min incubation in proteinase K, tissues were blocks with a solution containing 10% serum and 3% BSA. Primary antibodies were then incubated overnight at 4°C followed by incubation with an Alexa488 secondary antibody for 1h. Cell nuclei were labelled using Dapi.

### 6- Human subject and sample preparation

Informed consent was obtained from the parents of the patients prior to sample collections. Both patient PBMCs importation and iPSC derivation were approved by the Ministry of Higher Education, Research and Innovation (approval # IE-2019-1071 for importation and # DC-2019-3551 for iPSCs generation).

### 7- Induced Pluripotent Stem cells (iPSCs) generation and characterization

Peripheral-blood mononuclear cells (PBMCs) were isolated using whole blood from one SEDC patient carrying the G897R mutation and two unrelated individuals (COL2^{+/+}) using classical FICOLL gradient method. After 24 hours of recovery in StemPro-34 SFM medium (Invitrogen) supplemented with 100 ng/ml Stem Cell Factor (SCF, Peprotech), 100 ng/ml Fms-related tyrosine kinase 3 ligand (Flt3L, Peprotech), 20 ng/ml Interleukin-3 (IL-3, Peprotech) and 20 ng/ml IL-6 (Peprotech), 0.5 million PBMCs were processed using the CytoTune-iPS 2.0 Sendai Reprogramming Kit (Invitrogen), following manufacturers instruction, for iPSCs derivation.

Characterization of the iPSCs has been conducted as previously described (13). COL2^{+/+} and G897R iPSCs were assessed for their expression of stem cells markers OCT4, SOX2, SSEA4 and TRA-1-60 by immunohistochemistry. After fixation in paraformaldehyde and permeabilization with saponin, cells were incubated 3 hours at room temperature with the primary antibodies. Secondary antibodies were Alexa 594 and Alexa 488. Nuclei were stained with NucBlue Fixed Cells Stain (Dapi).

Embryoid bodies (EBs) were formed as previously described (13). iPSCs were harvested and clumps were transferred to a low adherent 6 well plate in differentiation medium containing 80% DMEM-F12, 20% Knock out Serum Replacer (KSR, Invitrogen), 1 mM non-essential amino acids, 1 mM Penicillin/Streptomycin, and 100 µM 2-mercapthoethanol. The medium was changed daily. After 14 days of differentiation, cells were recovered for RNA extraction and subsequent qPCR analysis of markers of the ectoderm, endoderm, and mesoderm.

### 8- Mesenchymal Stem Cells (MSCs) differentiation and characterization

iPSCs were differentiated directly into MSCs as previously described (13). Briefly, medium was replaced by MSC-inducing medium containing DMEM-F12, 10% fetal bovine serum (FBS), 1 mM sodium pyruvate, 1 mM Penicillin/Streptomycin, 1 mM non-essential amino acid, 110 µM 2-mercaptoethanol and 10 ng/mL Epidermal Growth Factor (EGF). The medium was changed every two days. After one week, cells were recovered using 0.25% trypsin-EDTA and transferred in tissue culture dish during one additional week. At this time, cells were harvested, phenotypically characterized by flow cytometry for their expression of MSC markers and assayed for their differentiation ability.

### 9- Adipocytes, osteoblasts and chondrocytes differentiation

Adipogenesis was investigated using the StemPro adipogenesis differentiation kit (Life Technologies). MSCs were seeded at 5×10⁴ per well, in a 24-well plate, and cultured for 2-3 weeks, in complete adipogenesis differentiation medium. Lipid deposits were observed following staining with Oil Red O (Sigma Aldrich), according to the manufacturer's instructions. Cells were fixed in 4% PFA for 10 min at room temperature, incubated 5 min in 60% isopropanol and finally transferred to an Oil Red O. solution for 5 min. After washing, cells were counterstained in Mayer's hematoxylin.

Chondrogenic differentiation was performed using the StemPro chondrogenesis differentiation kit (Life Technologies). Cells were first seeded in a 12-well plate, in aggregates containing 8×10⁴ cells, in 5 µL of MSC medium, and placed in a 37°C, 5% CO₂ incubator for one hour. Complete chondrogenic medium was then add, and the cells were cultured for 20 days. The medium was changed once a week. Chondrogenic matrix formation was observed following Alcian blue, Safranin O and Toluidine Blue staining.

For osteoblast differentiation, MSCs were plated in tissue culture 12 well plates (5×10⁵ cells/well) in a complete StemPro osteogenesis differentiation kit (Life Technologies) during 14 days. At the end of the differentiation, the presence of mineralized nodules was assessed using Alizarin Red S, Von Kossa (silver nitrate) and Alkaline Phosphatase staining.

Alizarin Red S., Von Kossa, Alcian Blue and Toluidine Blue staining were performed as previously described (13). Cells were first fixed in 4% PFA at room temperature during 15 minutes. After washing, cells were incubated either in a 1% Alizarin Red S., 1% Silver nitrate, 0.1% Toluidine Blue, 0.02% Alcian Blue or 0.1% Safranine O solution. For Von Kossa staining, cells were exposed to light until dark staining appears.

Alkaline phosphatase activity was measured using the leukocyte alkaline phosphatase staining kit (SIGMA), following the manufacturer's instructions. Cells were first fixed in 4% PFA for 10 min at room temperature, washed, and stained with Fast Violet for 15 minutes, in the dark at room temperature and imaged.

### 10- Flow cytometry analysis

Cell suspensions of MSCs were prepared by a 0.25% trypsin/EDTA treatment, and suspended in FACS buffer solution consisting in PBS, 3% Bovine Serum Albumin (BSA). Approximately 2×10⁵ cells were incubated with the desired cell surface marker antibodies or isotype control, at 4°C for 15 min. Specific antibodies for CD90, CD73 and CD105 (BD Biosciences), and isotype control immunoglobulin IgG1 (BD Biosciences) were used for labelling. Antibodies were diluted in FACS buffer. After 3 washes in FACS buffer, samples were fixed in 0.4% paraformaldehyde, and then proceed using an Accuri C6+ cytometer (BD Biosciences) and analyzed using FlowJo software (FlowJo, LLC).

### 11- Statistics

Sample size and power calculation for each parameter were determined using the online calculator powerandsamplesize.com using the assumption that data follow normality, the significance level α equals to 0.05 and that the F-test shows equal variances. Statistical analysis was performed using GraphPad Prism 7.0 software. D'Agostino & Pearson normality test was performed to verify normality. For skeletal measurements that fulfilled normality and equal variance requirements, a one-way ANOVA with a Dunnett's multiple comparison test (95% confidence interval to compare all the different groups) was performed. For skeletal measurements data sets that did not fulfill normality and equal variance requirements, a Kruskall-Wallis test was performed. Mean values for each group were compared using the two-tailed Student's test for comparisons of two independent groups.

### Results

### Overexpression of collagen type II alone is sufficient to induce in vitro chondrogenesis

To determine the potential of using human type II collagen as a transgene to treat type II collagenopathies, the inventors first performed experiments using mesenchymal stem cells (MSCs). For this, cells were genetically modified to overexpress type II collagen under the control of the EF1α promoter. This promoter is used as a strong positive promoter. 3D cultures were then generated using the liquid overlay technic for 3 weeks. MSCs usually need chondrogenic medium to differentiate into chondrocytes. Interestingly, when genetically modified to overexpress type II collagen, under normal non chondrogenic medium, MSCs had the potential to differentiate into chondrocytes (data not shown), by the expression of type X collagen, a marker of chondrocyte terminal differentiation.

These data suggested that overexpression of type II collagen alone was sufficient to induce in vitro chondrogenesis.

### Overexpression of collagen type II accelerates the in vitro chondrogenesis differentiation process

When cultured under chondrogenic medium conditions, MSCs usually differentiate into chondrocyte in approximately 2 to 3 weeks. When genetically modified to overexpress type II collagen, MSC differentiated faster into chondrocytes as seen by the presence of proteoglycan in the matrix and the expression of type X collagen as soon as day 6 post induction (Figure 1).

### Overexpression of collagen type !! restores bone growth in femur explants from homozygous SEDC fetal mice

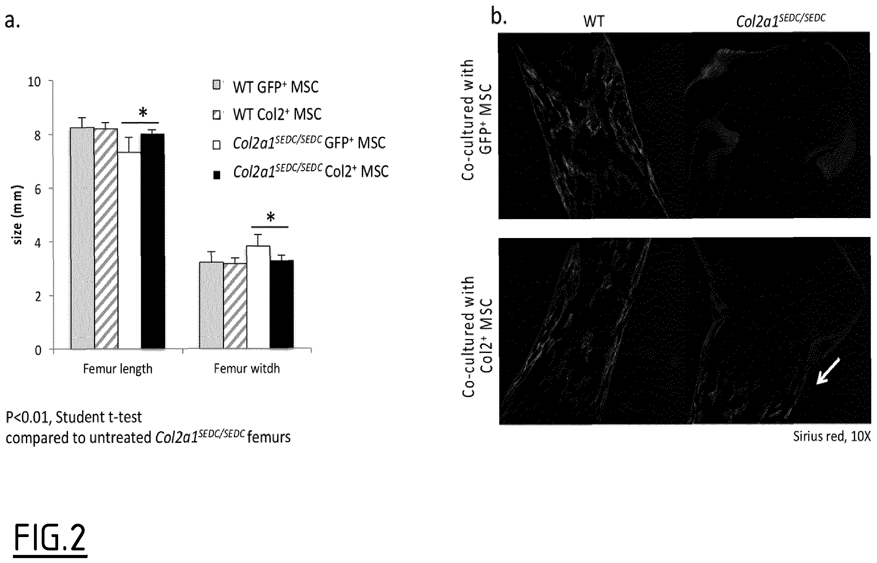

To determine the possibility of using type II collagen overexpression as a potential therapy for type II collagenopathies, femur explants from 16.5 dpc fetal homozygous Col1a1^{sedc/sedc} mice were co-cultured with MSCs overexpressing Col2 or GFP as a control. After 5 days in culture, explants were X-rayed and length and width were measured. As seen in Figure 2, the co-culture with MSCs expressing type II collagen resulted in a restoration of both length and width of the femoral bones. Proper bone mineralization was also observed as seen by the presence of collagen fibers in the diaphysis of the treated homozygous bones.

### Four constructs have high and specific expression in chondrocytes

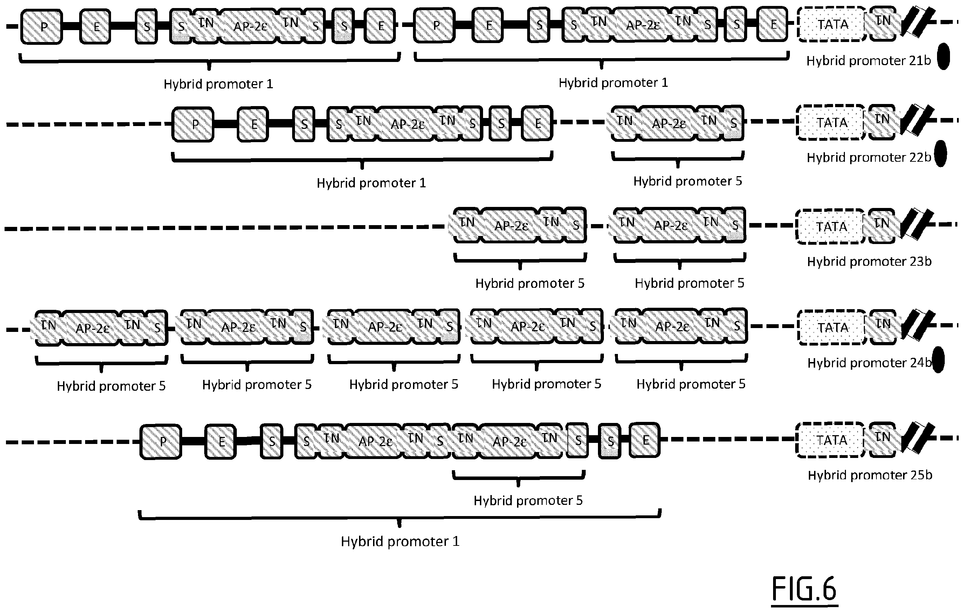

These preliminary results showing that overexpression of normal collagen type II induced normal chondrogenic differentiation of MSCs isolated from SEDC mice were performed using a retroviral vector under the control of the EF1α promoter. The EF1α promoter was initially chosen as it drives high levels of transgene expression allowing for a rapid screening of therapeutic efficacy. To limit unwanted side effects caused by ectopic type II collagen expression, the inventors created several chimeric promoters, allowing both for strong and tissue specific expression. To do this, the inventors have created 24 hybrid sequences between the transcription factor binding sequences of both the EF1α and the hCOL2A1 promoter sequences, using the EF1α backbone with regulatory elements of the hCOL2A1 promoter, or vice versa (Figures 3-6).

When designing the 24 constructs, the inventors that the core promoter sequence is between the upstream enhancer and the TATA box (which corresponds to the initiation of transcription).

Thus, it was expected that the use of the hEF1α core promoter would lead to higher expression levels, and that reversely, the use of the hCOL2A1 core promoter would lead to lower expression levels. Likewise, it was expected that the use of the AP2ε site would confer specificity, and that this would be even particularly true in the case of promoters comprising multiple tandems (such as promoters 23a, 23b and 25b).

Finally, the "b" promoter sequences, which contain additional N1 elements as compared to their "a" versions, were expected to show lower expression levels.

Thus, the 24 promoter constructs which were envisaged were as follows:
- promoters 1a and 1b of SEQ ID NO:18 and 19, respectively;
- promoters 5a and 5b of SEQ ID NO:20 and 21, respectively;
- promoters 11a and 11b of SEQ ID NO:22 and 23, respectively;
- promoters 12a and 12b of SEQ ID NO:24 and 25, respectively;
- promoters 13a and 13b (both are hybrid promoters according to the invention) of SEQ ID NO: 11 and 12, respectively;
- promoters 14a and 14b (both are hybrid promoters according to the invention) of SEQ ID NO: 13 and 14, respectively;
- promoters 15a and 15b (both are hybrid promoters according to the invention) of SEQ ID NO: 15 and 16, respectively;
- promoters 21a and 21b;
- promoters 22a and 22b (only 22a is a hybrid promoter according to the invention; it has SEQ ID NO:17);
- promoters 23a (of SEQ ID NO:26) and 23b (of SEQ ID NO:30);
- promoters 24a (of SEQ ID NO:27) and 24b; and
- promoters 25a and 25b of SEQ ID NO:28 and 29, respectively.

Among these 24 hypothetical constructs, it resulted that the seven promoters 1a, 21a, 24a, 25a, 21b, 22b and 24b were not possible to generate due to highly repetitive sequences (see Figures 3-6, the constructs with a point).

Expression levels obtained with each construct were evaluated by FACS analysis following transduction in chondrocyte-like cells (ATDC5, murine chondrocytic cell line). Expression in Baf3 cells (murine B cell line) was used to verify specificity. The constructs contained an IRES element to co-express collagen type II and GFP.

The following results were obtained:
- four constructs (13a, 13b, 14a and 14b) had high expression in chondrocytes with no expression in type B lymphocytes (Figure 7);
- the "b" promoter sequences showed lower levels of expression than their "a" versions;
- surprisingly, promoter sequences based on the hCOL2A1 core promoter showed the highest specificity. Especially, very good results were obtained with hybrid promoters 13a and 13b, which contain the regulatory elements of the EF1α promoter (EFP1 and EFP2 elements). Inversely, it was surprising that promoters 15a and 15b behave differently from hybrid promoters 13a, 13b, 14a and 14b, but these promoters 15a and 15b showed expression in B lymphocytes;
- hybrid promoters 13a, 13b, 14a and 14b surprisingly gave the highest expression levels, whereas they are based mainly on the hCOL2A1 promoter sequence;
- finally, as promoters 22-25 (in their "a" and "b" versions), which contain tandem repeats of hCOL2A1 core promoter, were expected to give high levels of expression, opposite results were obtained.

In view of these results, hybrid promoter 13a was chosen for subsequent evaluation in mice. A biodistribution study performed in newborn WT animals showed that, when normalized to the integration levels, expression levels driven by construct 13a are correlated to endogenous collagen type 2 expression levels, resulting in higher relative levels in growth plate cartilage (Figure 11).

### In vivo proof-of-concept in Col2^{sedc/sedc} mice

The number of animals included in the study was as follows:

| Group | Dose (ip/ml) | Genotype | *n* D1 | *n* D21 |
|---|---|---|---|---|
| Control | 0 | WT | 29 | 29 |
| | | HE | 54 | 51 |
| | | HO | 17 | 10 |
| Low | 7.5×10⁴ | WT | 17 | 16 |
| | | HE | 28 | 26 |
| | | HO | 10 | 6 |
| Medium | 2.4×10⁵ | WT | 9 | 9 |
| | | HE | 27 | 26 |
| | | HO | 9 | 8 |
| | | Total 200 | | 181 |

Following injection of a low dose of lentiviral particles of the hybrid promoter 13a according to the invention, at day 1 and day 7, the first observation was a significant correction of survival in treated homozygous animals compared to vehicle treated mice (Figure 8).

This increased survival was associated with a restoration of the rib cage volume and of the foramen magnum area, as described in the tables below and as shown in Figure 12. The distance between the foramen and the edge of the skull was also restored. These findings are important since the position of the skull is corrected following treatment and could protect patients for cervical instability.

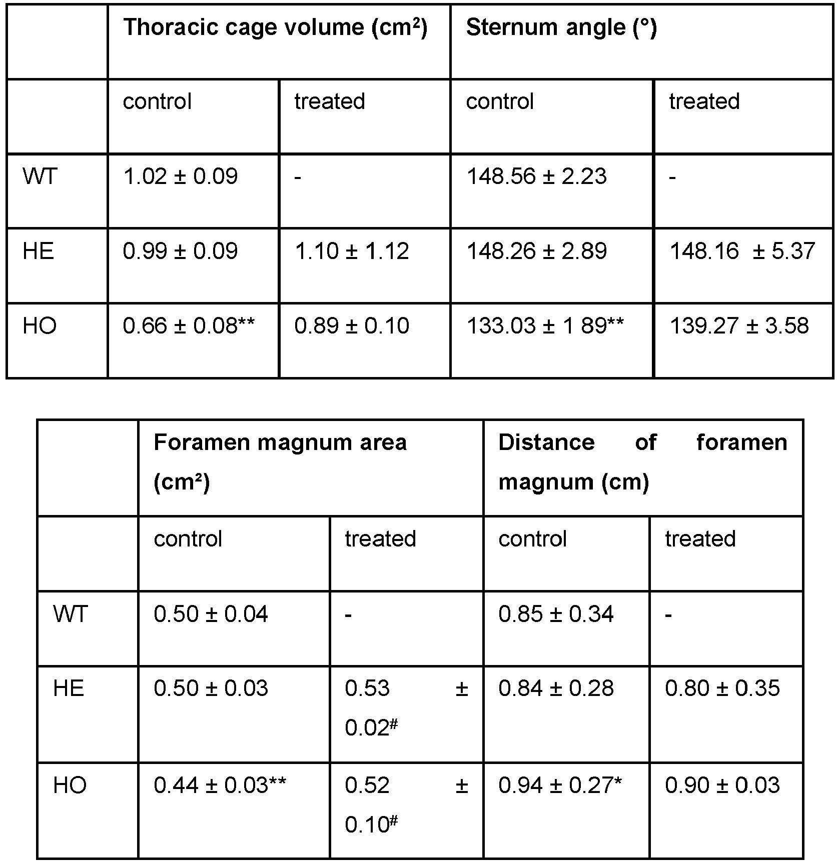

A dose response effect was observed on body and tail lengths as well as on the main long bones including humerus, tibia and femurs (Figure 10). This confirms a homogeneous effect of treatment restoring both the axial and the appendicular skeleton. Moreover, histological analysis showed that the architecture of the growth plate was restored in treated vs untreated HO mice (Figure 13) and that the collagen type 2 contents were increased in treated animals (data not shown).

### Generation of iPSCs from Sedc patients

To test the lentiviral vector-based therapy in an human context, the inventors used patient PBMCs to generate iPSCs. Genome analysis revealed that the patient carried the COL2A1 G897R variant, which is associated with Sedc. Induced pluripotent stem cells (iPSCs) were generated from PBMCs obtained from whole blood samples from a Sedc patient and two unrelated wild-type individual. After isolation, the cells were infected with Sendai virus containing the reprogramming factor OCT4, SOX2, KLF4 and c-MYC. Twenty one days after the infection, cells with an embryonic stem cell morphology were individually picked and expanded to obtain a clonal iPSCs population.

After several passages in culture, control (hereafter COL2^{+/+}) and Sedc (hereafter G897R) iPSCs displayed an embryonic stem cells morphology, indicating that the cells were able of self-renewal. This was verified by the expression of embryonic markers. iPSCs from control and from the patient both express OCT4, SOX2, SSEA4 and TRA-1-60 (Figure 14A), as determined via immunofluorescence. Their pluripotency was evaluated by their ability to form embryoid bodies (EBs) with differentiated endoderm, mesoderm and ectoderm layers. EBs were formed from COL2^{+/+} and G897R iPSCs by suspension culture for 14 days. Morphologically, no differences were observed in the EB formation between COL2^{+/+} and G897R iPSCs clones (Figure 14A).

For all clones, expression of the pluripotency markers OCT4 and *NANOG* significantly decreased (p<0.05) in EBs derived from COL2^{+/+} and G897R iPSCs when compared to undifferentiated iPSCs (Figure 14B). Expression of the endodermal genes, alpha-fetoprotein (*AFP*), GATA binding protein 4 (*GATA4*), and hepatocyte nuclear factor-3-beta (*HNF-3B*, *FOXA2*), increased following differentiation in COL2^{+/+} and G897R iPSCs relative to undifferentiated iPSCs (p<0.05) (Figure 14B). Expression of the mesodermal genes, *BRACHYURY,* Runt-related transcription factor 1 (*RUNX1*) and CD34, also exhibited a significant upregulation (p<0.05) after 14 days of differentiation (Figure 14B). Finally, expression of the ectodermal markers, *NESTIN,* Glial fibrillary acidic protein (*GFAP*), and sex determining region Y-box 1 (SOX1), displayed a similar increase in expression (p<0.05) in COL2^{+/+} and G897R iPSCs compared to undifferentiated iPSCs (Figure 14B). They also displayed strong alkaline phosphatase activity, characteristic of embryonic stem cells (Figure 14A).

In conclusion no differences were observed in levels of expression in these markers between EBs derived from iPSCs of all genotypes. These results indicate that COL2A1 is not genetically required for the formation of the three functional primordial germ layers.

### Treatment effects on Sedc patient cells

To evaluate treatment effect on patient cells, mesenchymal stem cells (MSCs) were differentiated from the previously generated iPSCs. Indeed, during the embryonic development, skeletal elements including bone and cartilage are derived from the mesenchymal lineage.

During the differentiation process, cells from both genotypes were able to undergo morphological changes indicating that the cells were differentiating. Whereas COL2^{+/+} cells displayed a typical MSCs organization (Figure 15A), the inventors observed that G897R cells were not able to have a proper MSCs organization (Figure 15A). This was corrected by the gene therapy as seen in Figure 15A. At the end of the differentiation, expression of typical MSC surface markers was assessed by FACS analysis. COL2^{+/+} cells were positive for CD90, CD105 and CD73 as expected. A shift in the cytometry peak was observed in the untreated patients with a significant decrease in all cell surface markers (Figure 15B). Following the genetic modification of only 10-15% of cells, the inventors observed a restoration of the mesenchymal cell surface markers for nearly the complete population, suggesting that only a few percentage of cells overexpressing normal type 2 collagen was sufficient to restore the organization of the mesenchymal network.

To go further, the inventors have evaluated the COL2^{+/+}, G897R and treated G897R MSCs differentiation potential into adipocytes, osteoblasts and chondrocytes. In all groups, cells demonstrated the same ability to differentiate into adipocytes (as shown by the Oil Red O. staining) and osteoblasts (proved by the positive Alizarin Red S, Von Kossa and strong alkaline phosphatase activity, data not shown). However, when submitted to chondrogenic differentiation, G897R MSCs present a lower chondrocytes differentiation ability compare to COL2^{+/+} MSCs, as shown by the reduced Alcian Blue, Toluidine Blue and Safranin O. staining (Figure 16), suggesting that loss of COL2A1 function impacted the chondrogenic extracellular matrix formation. After gene transfer, adipocyte and osteoblast differentiation abilities were not affected. However, treated patient cells displayed an increased chondrogenic differentiation ability, as shown by the increase in the Alcian Blue, Toluidine Blue and Safranine O. staining, compare to non-treated patient cells (Figure 16).

### Conclusion

- **the hybrid promoters 13a, 13b, 14a and 14b of the invention show increased levels of expression in chondrocytes, while being tissue-specific;**
- **the overexpression of type !! collagen restores bone growth in femur explants from homozygous SEDC fetuses;**
- **there is a potential for developing a direct gene therapy for the treatment of SEDC, and a tool for treating numerous cartilage diseases;**
- **treatment effect was also observed on the differentiation of mesenchymal stem cells derived from iPSCs obtained from patient cells. Interestingly, only a few percent of cells expressing normal type 2 collagen was sufficient to restore a near normal differentiation.**

### References

1. Spranger J, Brill P, Hall C, Nishimura G, Superti-Furga A, Unger S. Bone dysplasia - an atlas of genetic disorders of skeletal development (fourth edition). Oxford University Press. 2018.
2. Bonafe L, Cormier-Daire V, Hall C, Lachman R, Mortier G, Mundlos S, et al. Nosology and classification of genetic skeletal disorders: 2015 revision. Am J Med Genet A. 2015;167A(12):2869-92.
3. Jobling R, D'Souza R, Baker N, Lara-Corrales I, Mendoza-Londono R, Dupuis L, et al. The collagenopathies: review of clinical phenotypes and molecular correlations. Curr Rheumatol Rep. 2014;16(1):394.
4. Spranger J, Winterpacht A, Zabel B. The type II collagenopathies: a spectrum of chondrodysplasias. Eur J Pediatr. 1994;153(2):56-65.
5. Carter EM, Raggio CL. Genetic and orthopedic aspects of collagen disorders. Curr Opin Pediatr. 2009;21(1):46-54.
6. Terhal PA, Nievelstein RJ, Verver EJ, Topsakal V, van Dommelen P, Hoornaert K, et al. A study of the clinical and radiological features in a cohort of 93 patients with a COL2A1 mutation causing spondyloepiphyseal dysplasia congenita or a related phenotype. Am J Med Genet A. 2015;167A(3):461-75.
7. Terhal PA, van Dommelen P, Le Merrer M, Zankl A, Simon ME, Smithson SF, et al. Mutation-based growth charts for SEDC and other COL2A1 related dysplasias. Am J Med Genet C Semin Med Genet. 2012;160C(3):205-16.
8. Kosher RA, Kulyk WM, Gay SW. Collagen gene expression during limb cartilage differentiation. J Cell Biol. 1986;102(4):1151-6.
9. Gentili C, Cancedda R. Cartilage and bone extracellular matrix. Curr Pharm Des. 2009; 15(12):1334-48.
10. Nishimura G, Haga N, Kitoh H, Tanaka Y, Sonoda T, Kitamura M, et al. The phenotypic spectrum of COL2A1 mutations. Hum Mutat. 2005;26(1):36-43.
11. Savarirayan R, Bompadre V, Bober MB, Cho T-J, Goldberg MJ, Hoover-Fong J, et al. Best practice guidelines regarding diagnosis and management of patients with type II collagen disorders. Genetics in Medicine. 2019.
12. Okada M, Ikegawa S, Morioka M, Yamashita A, Saito A, Sawai H, et al. Modeling type II collagenopathy skeletal dysplasia by directed conversion and induced pluripotent stem cells. Hum Mol Genet. 2015;24(2):299-313.
13. Pini J, Rouleau M, Desnuelle C, Sacconi S, Bendahhou S. Modeling Andersen's Syndrome in Human Induced Pluripotent Stem Cells. Stem Cells Dev. 2016;25(2):151-9.

## Claims

1. Promoter comprising the nucleic acid sequence SEQ ID NO:1, in which a fragment starting from position A and ending at position B of said sequence SEQ ID NO:1 has been substituted by a fragment of the hEF1α promoter comprising at least the TATA box,
wherein position A is comprised between nucleotides 521 and 555 of SEQ ID NO:1, and position B is comprised between nucleotides 560 and 587 of SEQ ID NO:1, and wherein the fragment consisting of nucleotides 598 to 607 of SEQ ID NO:1 may be present or absent, wherein the fragment of the hEF1α promoter comprising at least the TATA box is chosen from sequences SEQ ID NO:2, 3 and 4.

2. Promoter according to claim 1, wherein the fragment starting from nucleotide 541 and ending at nucleotide 560 of sequence SEQ ID NO:1 has been substituted by the fragment of the hEF1α promoter comprising at least the TATA box of sequence SEQ ID NO:2, and
the fragment consisting of nucleotides 598 to 607 of SEQ ID NO:1 is absent.

3. Promoter according to claim 1, wherein the fragment starting from nucleotide 541 and ending at nucleotide 560 of sequence SEQ ID NO:1 has been substituted by the fragment of the hEF1α promoter comprising at least the TATA box of sequence SEQ ID NO:2, and
the fragment consisting of nucleotides 598 to 607 of SEQ ID NO:1 is present.

4. Promoter according to claim 1, wherein the fragment starting from nucleotide 555 and ending at nucleotide 560 of sequence SEQ ID NO:1 has been substituted by the fragment of the hEF1α promoter comprising at least the TATA box of sequence SEQ ID NO:3, and
the fragment consisting of nucleotides 598 to 607 of SEQ ID NO:1 is absent.

5. Promoter according to claim 1, wherein the fragment starting from nucleotide 555 and ending at nucleotide 560 of sequence SEQ ID NO:1 has been substituted by the fragment of the hEF1α promoter comprising at least the TATA box of sequence SEQ ID NO:3, and
the fragment consisting of nucleotides 598 to 607 of SEQ ID NO:1 is present.

6. Promoter according to claim 1, wherein the fragment starting from nucleotide 521 and ending at nucleotide 587 of sequence SEQ ID NO:1 has been substituted by the fragment of the hEF1α promoter comprising at least the TATA box of sequence SEQ ID NO:4, and
the fragment consisting of nucleotides 598 to 607 of SEQ ID NO:1 is absent.

7. Promoter according to claim 1, wherein the fragment starting from nucleotide 521 and ending at nucleotide 587 of sequence SEQ ID NO:1 has been substituted by the fragment of the hEF1α promoter comprising at least the TATA box of sequence SEQ ID NO:4, and
the fragment consisting of nucleotides 598 to 607 of SEQ ID NO:1 is present.

8. Vector comprising the promoter according to any one of claims 1 to 7.

9. Vector according to claim 8, in which the promoter controls the expression of a gene or a fragment thereof coding for a collagen chain, preferably COL2A1.

10. Promoter according to any one of claims 1 to 7, or vector according to claim 8 or 9, for use in therapy.

11. Promoter according to any one of claims 1 to 7, or vector according to claim 8 or 9, for use in gene therapy.

12. Promoter according to any one of claims 1 to 7, or vector according to claim 8 or 9, for use for treating a skeletal dysplasia or a disease of the musculoskeletal system.

13. Promoter according to any one of claims 1 to 7 or 12, or vector according to claim 8 or 9 or 12, for use for treating a type II collagenopathy.

14. Promoter for use according to claim 13, or vector for use according to claim 13, wherein the type II collagenopathy is spondyloepiphyseal dysplasia congenital (SEDC).

## Patentansprüche

1. Promotor, umfassend die Nucleinsäuresequenz SEQ ID Nr. 1, bei dem ein von Position A beginnendes und bei Position B der Sequenz SEQ ID Nr. 1 endendes Fragment durch ein mindestens die TATA-Box umfassendes Fragment des hEFla-Promotors ersetzt worden ist,
wobei Position A zwischen den Nucleotiden 521 und 555 von SEQ ID Nr. 1 enthalten ist, und Position B zwischen den Nucleotiden 560 und 587 von SEQ ID Nr. 1 enthalten ist, und wobei das aus den Nucleotiden 598 bis 607 von SEQ ID Nr. 1 bestehende Fragment vorhanden sein oder fehlen kann, wobei das mindestens die TATA-Box umfassende Fragment des hEFla-Promotors aus den Sequenzen SEQ ID Nr. 2, SEQ ID Nr. 3 und SEQ ID Nr. 4 ausgewählt ist.

2. Promotor nach Anspruch 1, wobei das von Nucleotid 541 beginnende und bei Nucleotid 560 von Sequenz SEQ ID Nr. 1 endende Fragment durch das mindestens die TATA-Box umfassende Fragment des hEFla-Promotors von Sequenz SEQ ID Nr. 2 ersetzt worden ist, und
das aus den Nucleotiden 598 bis 607 von SEQ ID Nr. 1 bestehende Fragment fehlt.

3. Promotor nach Anspruch 1, wobei das von Nucleotid 541 beginnende und bei Nucleotid 560 von Sequenz SEQ ID Nr. 1 endende Fragment durch das mindestens die TATA-Box umfassende Fragment des hEFla-Promotors von Sequenz SEQ ID Nr. 2 ersetzt worden ist, und
das aus den Nucleotiden 598 bis 607 von SEQ ID Nr. 1 bestehende Fragment vorhanden ist.

4. Promotor nach Anspruch 1, wobei das von Nucleotid 555 beginnende und bei Nucleotid 560 von Sequenz SEQ ID Nr. 1 endende Fragment durch das mindestens die TATA-Box umfassende Fragment des hEFla-Promotors von Sequenz SEQ ID Nr. 3 ersetzt worden ist, und
das aus den Nucleotiden 598 bis 607 von SEQ ID Nr. 1 bestehende Fragment fehlt.

5. Promotor nach Anspruch 1, wobei das von Nucleotid 555 beginnende und bei Nucleotid 560 von Sequenz SEQ ID Nr. 1 endende Fragment durch das mindestens die TATA-Box umfassende Fragment des hEFla-Promotors von Sequenz SEQ ID Nr. 3 ersetzt worden ist, und
das aus den Nucleotiden 598 bis 607 von SEQ ID Nr. 1 bestehende Fragment vorhanden ist.

6. Promotor nach Anspruch 1, wobei das von Nucleotid 521 beginnende und bei Nucleotid 587 von Sequenz SEQ ID Nr. 1 endende Fragment durch das mindestens die TATA-Box umfassende Fragment des hEFla-Promotors von Sequenz SEQ ID Nr. 4 ersetzt worden ist, und
das aus den Nucleotiden 598 bis 607 von SEQ ID Nr. 1 bestehende Fragment fehlt.

7. Promotor nach Anspruch 1, wobei das von Nucleotid 521 beginnende und bei Nucleotid 587 von Sequenz SEQ ID Nr. 1 endende Fragment durch das mindestens die TATA-Box umfassende Fragment des hEFla-Promotors von Sequenz SEQ ID Nr. 4 ersetzt worden ist, und
das aus den Nucleotiden 598 bis 607 von SEQ ID Nr. 1 bestehende Fragment vorhanden ist.

8. Vektor, umfassend den Promotor nach einem der Ansprüche 1 bis 7.

9. Vektor nach Anspruch 8, in dem der Promotor die Expression eines eine Kollagenkette codierenden Gens oder Fragments davon, vorzugsweise COL2A1, reguliert.

10. Promotor nach einem der Ansprüche 1 bis 7 oder Vektor nach Anspruch 8 oder 9, zur Verwendung in der Therapie.

11. Promotor nach einem der Ansprüche 1 bis 7 oder Vektor nach Anspruch 8 oder 9, zur Verwendung in der Gentherapie.

12. Promotor nach einem der Ansprüche 1 bis 7 oder Vektor nach Anspruch 8 oder 9, zur Verwendung zur Behandlung einer Skelettdysplasie oder einer Erkrankung des Stütz- und Bewegungsapparates.

13. Promotor nach einem der Ansprüche 1 bis 7 oder 12 oder Vektor nach Anspruch 8 oder 9 oder 12, zur Verwendung zur Behandlung einer Typ-II-Kollagenopathie.

14. Promotor zur Verwendung nach Anspruch 13 oder Vektor zur Verwendung nach Anspruch 13, wobei die Typ-II-Kollagenopathie kongenitale spondyloepiphysäre Dysplasie (SEDC) ist.

## Revendications

1. Promoteur comprenant la séquence d'acide nucléique SEQ ID n° 1, dans lequel un fragment débutant à la position A et se terminant à la position B de ladite séquence SEQ ID n° 1 a été substitué par un fragment du promoteur hEF1α comprenant au moins la boîte TATA,
dans lequel la position A est comprise entre les nucléotides 521 et 555 de SEQ ID n° 1 et la position B est comprise entre les nucléotides 560 et 587 de SEQ ID n° 1 et dans lequel le fragment constitué des nucléotides 598 à 607 de SEQ ID n° 1 peut être présent ou absent, dans lequel le fragment du promoteur hEF1α comprenant au moins la boîte TATA est choisi parmi les SEQ ID n° 2, 3 et 4.

2. Promoteur selon la revendication 1, dans lequel le fragment débutant au nucléotide 541 et se terminant au nucléotide 560 de la séquence SEQ ID n° 1 a été substitué par le fragment du promoteur hEF1α comprenant au moins la boîte TATA de la séquence SEQ ID n° 2 et
le fragment constitué de nucléotides 598 à 607 est absent.

3. Promoteur selon la revendication 1, dans lequel le fragment débutant au nucléotide 541 et se terminant au nucléotide 560 de la séquence SEQ ID n° 1 a été substitué par le fragment du promoteur hEF1α comprenant au moins la boîte TATA de la séquence SEQ ID n° 2 et
le fragment constitué des nucléotides 598 à 607 de SEQ ID n° 1 est présent.

4. Promoteur selon la revendication 1, dans lequel le fragment débutant au nucléotide 555 et se terminant au nucléotide 560 de la séquence SEQ ID n° 1 a été substitué par le fragment du promoteur hEF1α comprenant au moins la boîte TATA de la séquence SEQ ID n° 3 et
le fragment constitué des nucléotides 598 à 607 de SEQ ID n° 1 est absent.

5. Promoteur selon l
a revendication 1, dans lequel le fragment débutant au nucléotide 555 et se terminant au nucléotide 560 de la séquence SEQ ID n° 1 a été substitué par le fragment du promoteur hEF1α comprenant au moins la boîte TATA de la séquence SEQ ID n° 3 et
le fragment constitué des nucléotides 598 à 607 de SEQ ID n° 1 est présent.

6. Promoteur selon la revendication 1, dans lequel le fragment débutant au nucléotide 521 et se terminant au nucléotide 587 de la séquence SEQ ID n° 1 a été substitué par le fragment du promoteur hEF1α comprenant au moins la boîte TATA de la séquence SEQ ID n° 4 et
le fragment constitué des nucléotides 598 à 607 de SEQ ID n° 1 est absent.

7. Promoteur selon la revendication 1, dans lequel le fragment débutant au nucléotide 521 et se terminant au nucléotide 587 de la séquence SEQ ID n° 1 a été substitué par le fragment du promoteur hEF1α comprenant au moins la boîte TATA de la séquence SEQ ID n° 4 et
le fragment constitué des nucléotides 598 à 607 de SEQ ID n° 1 est présent.

8. Vecteur comprenant le promoteur selon l'une quelconque des revendications 1 à 7.

9. Vecteur selon la revendication 8, dans lequel le promoteur contrôle l'expression d'un gène ou d'un de ses fragments codant pour une chaîne de collagène, de préférence COL2A1.

10. Promoteur selon l'une quelconque des revendications 1 à 7, ou vecteur selon la revendication 8 ou 9, destiné à être utilisé en thérapie.

11. Promoteur selon l'une quelconque des revendications 1 à 7, ou vecteur selon la revendication 8 ou 9, destiné à être utilisé en thérapie génique.

12. Promoteur selon l'une quelconque des revendications 1 à 7 ou vecteur selon la revendication 8 ou 9, destiné à être utilisé pour le traitement d'une dysplasie squelettique ou d'une maladie du système musculosquelettique.

13. Promoteur selon l'une quelconque des revendications 1 à 7 et 12 ou vecteur selon la revendication 8 ou 9 ou 12, destiné à être utilisé pour le traitement d'une collagénopathie de type II.

14. Promoteur destiné à être utilisé selon la revendication 13 ou vecteur destiné à être utilisé selon la revendication 13, dans lequel la collagénopathie de type II est la dysplasie spondyloépiphyséale congénitale (SEDC).
